# EUROPEAN PATENT SPECIFICATION

(11) **EP 2 249 802 B1**
(45) Date of publication and mention of the grant of the patent: **16.01.2013**
(21) Application number: 09708982.5
(22) Date of filing: 06.02.2009
(51) Int. Cl.: A61K 9/00, A61K 9/06, A61K 47/38, A61K 47/42

(54) **COMPOSITION AND METHOD FOR ASSISTING SWALLOWING**
ZUSAMMENSETZUNG UND VERFAHREN ZUR UNTERSTÜTZUNG DES SCHLUCKVORGANGS
COMPOSITION ET PROCÉDÉ D'AIDE À LA DÉGLUTITION

(30) Priority: 06.02.2008 US 26577
(43) Date of publication of application: 17.11.2010
(73) Proprietor: University of East Anglia, Norwich, Norfolk NR4 7TJ (GB)
(72) Inventor: CRAIG, Duncan Q.M., Norwich Norfolk NR4 7TJ (GB); WRIGHT, David J., Norwich Norfolk NR4 7TJ (GB); MENCARELLI, Giovanna, Marche 64147 (IT); ROGERSON, Madeleine, Norwich Norfolk NR4 7TJ (GB)
(74) Representative: Rogers, Alex Lee
(86) International application number: PCT/GB2009/050123
(87) International publication number: WO 2009/098520

(56) References cited:
- EP-A- 0 722 721
- EP-A- 0 950 402
- EP-A1- 2 133 060
- US-A- 5 114 720
- DATABASE WPI Week 199726 Thomson Scientific, London, GB; AN 1997-285132 XP002537994 & JP 09 104621 A (BS KK) 22 April 1997 (1997-04-22)
- CHOW F ET AL: "La cuerda dulce - A tolerability and acceptability study of a novel approach to specimen collection for diagnosis of paediatric pulmonary tuberculosis" BMC INFECTIOUS DISEASES 20060404 GB, vol. 6, 4 April 2006 (2006-04-04), XP002536242 ISSN: 1471-2334 1471-2334
- WILSON C G ET AL: "A gamma scintigraphic study to compare oesophageal clearance of 'Expidet' formulations, tablets and capsules in supine volunteers", INTERNATIONAL JOURNAL OF PHARMACEUTICS 1988 NL LNKD- DOI:10.1016/0378-5173(88)90084-1, vol. 46, no. 3, 1988, pages 241-246, ISSN: 0378-5173
- DEUTSCHES PATENT- UND MARKENAMT: 'Informationen zum Geschmacksmuster M9802599-0001', [Online] 25 November 1998, Retrieved from the Internet: <URL:http://register.dpma.de/DPMAregister/g sm/register?GSNR=M9802599-0001> [retrieved on 2011-03-29]
- DEUTSCHES PATENT UND MARKENAMT: 'Informationen zum Geschmacksmuster M9709311-0001', [Online] 09 April 1998, Retrieved from the Internet: <URL:http://register.dpma.de/DPMAregister/g sm/register?GSNR=M9709311-0001> [retrieved on 2011-03-29]
- WRIGHT DAVID: "Medication administration in nursing homes.", NURSING STANDARD (ROYAL COLLEGE OF NURSING (GREAT BRITAIN) : 1987) 2002 JUL 3-9 LNKD- PUBMED:12216309, vol. 16, no. 42, 3 July 2002 (2002-07-03), pages 33-38, ISSN: 0029-6570

## Description

### Field of the Invention

The present invention relates to the treatment of a subject having difficulty in swallowing. In particular, the present invention relates to compositions that are easy to swallow and can be used to administer a solid that would otherwise be difficult to swallow, which is a dosage form such as a tablet or capsule, as defined by the claims.

The present disclosure also relates to preventing solid oral pharmaceutical dosage formulations from lodging in a patient's esophagus or sticking to the walls of a patient's esophagus.

### Background

A difficulty in swallowing prevents the easy passage of solids or liquids from the mouth to the stomach. Dysphagia is a general term that includes a difficulty in swallowing deriving from physical impairment. Dysphagia can accompany many disorders, including, but not limited to, blunt throat injury, surgery-caused impairment, stroke, multiple sclerosis, Asperger syndrome, esophageal cancer, laryngeal cancer, chagus disease, celiac, cystic fibrosis, Huntington's disease, Niemann-Pick disease, neurological conditions such as amyotrophic lateral sclerosis, Alzheimer's and Parkinson's disease, obesity, Riley-Day syndrome, high cholesterol, corn allergies and corn sensitivities, sclerodenna, and diabetes. A difficulty in swallowing is more commonly seen in the elderly, and need not necessarily be caused by or associated with any other disorder. Dysphagia with a physical cause can be classified into two main types: oropharyngeal dysphagia (which generally arises from abnormalities of the upper esophagus, pharynx and the oral cavity) and esophageal dysphagia (which generally arises from the body of the esophagus, lower esophageal sphincter, or cardia or the stomach).

Difficulty in swallowing may not have an identifiable physical cause; such dysphagia is sometimes termed functional dysphagia in the art. Additionally, the difficulty in swallowing may have a psychological or psychogenic cause; such difficulty in swallowing is sometimes termed phagophobia.

Anxiety about the substance to be swallowed, for example food or a medicament, can also cause difficulty in swallowing, for example in children.

The term "dysphagia" as used herein refers generally to difficulty in swallowing, of any cause. It includes, but is not limited to, difficulty in swallowing solids and/or liquids, such as water.

It is well documented that persons with dysphagia experience difficulty in swallowing conventional tablets and capsules, often leading to dosage forms being crushed or compromised in some way prior to administration that can lead to potential changes in their biological profile. Previous attempts have focused on liquid-like materials or simply the use of foods such as yoghurt or commercial jelly.

However, foodstuffs can interact with the medicines and can potentially affect their efficacy on administration. They may also be cumbersome to administer (co-administered foodstuffs would typically be largely liquid-like in texture) and are non-standardised both in terms of composition, quantity co-administered, and means by which the medicine is incorporated prior to administration, hence there may be considerable variability in the manner in which the medicines are given or taken on each administration.

Even in patients who do not suffer from dysphagia, certain solid oral pharmaceutical formulations can lodge in the patient's esophagus or stick to the walls of the patient's esophagus, causing inflammation or ulcers (Chisaka, H. et al, Dysphagia, 21(4), pages 275-279 (2006); Kikendall, J. W. et al, Digestive Diseases and Sciences, 28(2), pages - (1983)).

EP 0 722 721 A discloses food-like drugs comprising seamless capsules and at least one gel, like pudding or jelly (gelatin). The high viscous liquid/ jelly covers the seamless capsules and increases the smoothness of its surface for easy swallowing of the food-like medicine by children and sickly people.

The present invention is based on our finding that certain compositions can be used to provide a lubricating surface to solid edible items, e.g. oral pharmaceutical formulations, which serves to assist swallowing of the formulation into the stomach, both for patients with and without dysphagia. Further advantages of compositions of the present invention are described below.

### Summary of the Invention

According to a first aspect of the present invention, there is provided a method of enabling a subject, having difficulty in swallowing, to swallow an object, the method comprising providing on the object an at least partial surface covering comprising a lubricating edible gel. The subject may be selected from humans and animals. The object may be an edible object. The object may be an otherwise unswallowable object, which includes, but is not limited to, an object that the subject cannot swallow in the absence of the lubricating, edible gel.

In an embodiment, the present invention provides a method of enabling a person, having difficulty in swallowing, to swallow an otherwise unswallowable object, the method comprising providing on the object an at least partial surface covering comprising a lubricating edible gel.

In one embodiment, the method of the first or second aspects of the invention consists essentially of, for example consists of, providing on the object or solid oral pharmaceutical formulation an at least partial surface covering comprising a lubricating edible gel.

The methods of the present disclosure may be therapeutic methods or non-therapeutic methods.

T The surface covering consists essentially of, for example consists of, the lubricating edible gel. The gel may constitute all or part of the surface, and/or over all or part of the total surface area, and/or may be present through all or part of the thickness of the covering.

In any aspect of the present disclosure, the subject may be selected from humans and animals. The human subject may be a person of any age and may be male or female. In an example the human subject may be a child or infant. In an example, the human subject may be elderly. The human subject may have an age of from 0 years to 16 years, optionally from 0 to 10 years, optionally from 0 to 4 years. The human subject may have an age of 50 year or more, optionally 60 years or more, optionally 70 years or more, optionally 80 years or more.

In an example, the subject may be an animal. The animal may, for example, be a domestic pet, including, but not limited to, a dog, a cat or a small mammal, such as a gerbil or a hamster.

The object is a solid medicament ("dosage form"). Solid medicaments include tablets and capsules.

According to a second aspect of the present invention, there is provided a method of preventing lodging of a solid oral medicament in the esophagus of a patient or of preventing a solid oral medicament from sticking to the walls of the esophagus of a patient, the method comprising providing the solid oral pharmaceutical formulation with a lubricating edible gel surface.

According to a third aspect of the present invention, there is provided a solid medicament including a surface comprising a lubricating edible gel, wherein the surface area on which gel is present is sufficient to enable a subject (e.g a person or animal), having difficulty in swallowing, to swallow the item.

In one embodiment of this aspect, the surface consists essentially of, for example consists of, the lubricating edible gel. The gel may constitute all or part of the surface, and/or over all or part of the total surface area, and/or may be present through all or part of the item.

The expression "lubricating" used herein includes, but is not limited to, edible gels which, on wetting by contact with mouth or throat fluids, provide a lubricious surface to enable the gel and the object it is provided on to be swallowed. The compositions of the present invention may be administered with a liquid, e.g. water, or without a liquid. Saliva may or may not be present in the mouth of the subject. It is most preferred that the gel for use in the present invention will attract, and optionally at least partially dissolve or suspend in, mouth or throat fluids within a few (e.g. up to about 5) seconds at body temperature (c. 37 °C), to provide - on taking of the gel into the subject's (e.g. person's or animal's) mouth - a surface coating of lubricious fluid.

It follows that it is not essential that the lubricating edible gel is slippery in the dry state, although it may be.

Gels for use in the present disclosure include, for example, hydrocolloids such as natural gelling polysaccharides (for example, agar, alginate, gums such as gellan gum, glucomannan, locust bean gum, xanthan gum), synthetic gums, gelling proteins (for example gelatin, for example gelatin A, pectin), and mixtures thereof.

It is preferred that a lubricating agent such as a synthetic cellulose derivative is present in the gel to improve the gel's lubricating effect in the mouth and throat in use, as described in more detail below.

The gels may, if desired, be pleasantly flavoured to assist the swallowing process.

A gel covering for a solid object to be swallowed may be provided on the object by setting of the heated liquid pre-gel *in situ* on the object, preferably in a suitably shaped mould. In another embodiment, the gel may be pre-shaped and the shaped (solidified) gel brought into physical association with the object to provide the at least partial covering thereon. The shape may be a sheet adapted to be wrapped around the object, or may be a receptacle such as a pocket or bowl shape, provided that the object can be secured in association with the covering for the swallowing process, e.g. by push-fitting. The shape is preferably selected and dimensioned according to the intended use, i.e. the subject (e.g. person or class of persons; or animal or class of animals) and the object or class of objects to be swallowed.

The suitably selected and dimensioned shaped gel form is novel and constitutes a further aspect of the present disclosure.

Therefore, in a fourth aspect, the invention provides an edible gel composition for oral administration to a subject with difficulty in swallowing, wherein the composition is shaped and dimensioned (e.g. having a recess therein) for the acceptance of a dosage form.

The embodiments described above in relation to the first to third aspects of the invention apply equally to the fourth aspect.

An item of solid ingestible material to be swallowed, including a surface comprising a lubricating edible gel, may be formed in conventional manner for such items using the gel material as a coating or as a filler for all or a portion of the bulk of the item, provided that the surface of the item displays the gel according to the present invention.

The present disclosure is useful for people who suffer from difficulty in swallowing of any cause.

The edible gel preferably has sufficient rigidity at room temperature to enable a pre-formed solid object to be swallowed, for example a tablet or capsule, to be fitted, e.g. push-fitted, into physical association with a pre-formed receptacle therefor, the receptacle comprising the edible gel.

Certain compositions for use in the present invention are novel, and these constitute further aspects of the present invention.

In a fifth aspect of the present disclosure, there is provided a solid medicament form, for example a tablet or capsule, at least part of the surface of which comprises a lubricating edible gel comprising a hydrocolloid.

The embodiments described above in relation to the first, second, third and fourth aspects of the invention apply equally to the fifth aspect.

In a sixth aspect, the present disclosure provides an edible gel composition, wherein the composition comprises:
a gelling agent,
water, and
a lubricating agent;
and the composition optionally comprises a recess therein for acceptance of a dosage form.

In a seventh aspect, the present invention provides a method for preparing an edible gel composition for oral administration to a subject having difficulty in swallowing, the method comprising providing a liquid pre-gel composition for forming an edible gel; and then either:
(i) solidifying the pre-gel composition in a mould such that a recess for acceptance of a dosage form is formed in the solidified edible gel composition, or
(ii) solidifying the pre-gel composition in a mould and forming a recess in the solidified gel, e.g. by cutting a recess in the gel.

In an eighth aspect, the present disclosure provides a method for preparing an edible gel composition for oral administration to a subject with difficulty in swallowing, the method comprising providing a liquid pre-gel composition comprising
a gelling agent,
water, and
a lubricating agent; and
solidifying the pre-gel composition in a mould.

In a ninth aspect, the present invention provides a method of administering a dosage form to a subject having difficulty in swallowing, comprising
providing a composition according to any one of the fourth and sixth aspects of the present invention having a dosage form associated therewith; and
administering the composition orally to the subject.

In a tenth aspect, the present disclosure provides a method of treating a subject having difficulty in swallowing and optionally having another disorder, the method comprising administering orally a composition of the present invention having a dosage form associated with the composition, the dosage form being for the treatment of the difficulty in swallowing and/or the other disorder. The other disorder may be any disorder the subject has simultaneously with the difficulty in swallowing. It may be a disorder clinically associated with the difficulty in swallowing.

In an eleventh aspect, the present invention provides the use of a composition of the present invention in the manufacture of a medicament for the treatment of a subject having difficulty in swallowing and/or another disorder.

In a twelfth aspect, the present invention provides a package containing an edible composition of the present invention, optionally together with instructions for use according the present invention.

### Detailed Description of the Invention

The present invention provides the first to the eleventh aspects as described above.

"Difficulty in swallowing" includes, but is not limited to, dysphagia, such as oropharyngeal dysphagia and esophageal dysphagia, difficulty in swallowing without an identifiable physical cause and difficulty in swallowing having a psychogenic cause, such as phagophobia.

A "gel" includes, but is not limited to, a self-supporting, flexible substance formed from a liquid, e.g. water, and a gelling agent. Gels are known in physical chemistry and can include colloidal dispersions of a solid or gas within a liquid. Gels may be considered to be jelly-like substances. The gel is preferably conformable to or around a dosage form, such as a tablet or a capsule. The gel is preferably capable of melting, or dissolving or suspending in water, at an elevated temperature (e.g. from about 25 °C to about 90 °C) and setting on cooling to provide the said self-supporting flexible material at room temperature. The gel may be ingested by the user without adverse effects.

The subject or user of the invention may be a human or animal.

The composition of the present invention has surprising advantages over prior art methods of administering dosage forms to a subject with difficulty in swallowing. In particular, the gel is preferably a self-supporting, flexible substance. It can preferably be picked up and handled.. The gel is preferably water-miscible and the water-miscible nature of the gel preferably allows for some lubrication when inserted in the mouth cavity, thus easing swallowing of the gel composition by a subject with difficulty in swallowing. A lubricating agent is present in the gel. A lubricating agent includes, but is not limited to, an agent that increases the ease with which the composition will pass through a simulated swallowing testing device (as described in the examples), and hence the ease with which the composition can be swallowed by a subject with difficulty in swallowing, compared to an analogous composition (i.e. formed with the same gelling agent(s) in the absence of the lubricating agent and comprising the same total concentration of gelling agent(s)) that does not contain the lubricating agent. The present inventors have also found in *in vitro* studies that, for at least for some embodiments of the present invention, if a tablet is contained within a recess in the gel, the presence of the surrounding gel does not significantly slow the disintegration and/or dissolution of the tablet in an acid that is of a comparable strength to stomach acid. This indicates that the gels may not significantly (if at all) impede drug absorption.

Further, if a recess is provided in the composition, one can easily deform the gel composition and insert a dosage form, such as a tablet or capsule. The recess is preferably a slit or recess and is such that, when the dosage is inserted into the recess, it is held in place, to allow for placing the composition in a subject's mouth. The gel composition may fully encapsulate the dosage form or may retain the dosage form, for example in the recess, without full encapsulation. Optionally, an insert or slit divider may be provided such that, in storage before use, the slit is maintained and can be opened, once the insert/divider or removed, for insertion of a edible object, such as a dosage form. The slit divider may comprise a suitable material, such as a plastic or a metal. The slit divider may form part of a packaging in which the composition is stored. The slit divider may form part of a lid of the packaging.

The gelling agent and the lubricating agent are different materials. In a preferred embodiment, the gelling agent and lubricating agent are different materials, and examples of such compositions are given below.

The gelling agent is preferably a hydrocolloid gelling agent. The gelling agent may comprise a polysaccharide gelling agent. The gelling agent may be a protein-containing gelling agent, such as gelatin or pectin. Preferably, the gelling agent is selected from gelatin, hydryxypropylmethylcelluluse, alginates, chitosan, agar, carrageenan, pectin, gellan gum, glucomannan, locust bean gum, xanthan gum, and mixtures thereof. Most preferably, the gelling agent comprises gelatin. Gelatin has been found to be particularly suitable due to the high strength and rigidity of the gelatin gels at comparatively low concentrations. The gelatin may be selected from any type of gelatin, including, but not limited to, gelatin type A, gelatin type B, and mixtures thereof. The gelling agent may comprise gelatin type A, preferably derivable from porcine skin; such gelatin is available from commercial sources (e.g Sigma Aldrich®). Gelatin type B may be cow-derived and is available commercially from VWR (BHS). Mixtures of types A and B gelatin are available commercially from Sigma Aldrich. Preferably the gelatin has Ph Eur grading and/or BSE/TSE certificates. The gelling agent is preferably a water-soluble gelling agent. The gelling agent is preferably soluble in water (i.e. can form an aqueous solution) at temperature of 35 °C or above, preferably 50 °C or above, most preferably 70 °C at a concentration of from 1 to 3 % by weight. Preferably, an aqueous solution of the gelling agent forms a self-supporting, flexible gel on cooling to a temperature of 35 °C or less, preferably 30 °C or less, more preferably 25 °C or less. Preferably, an aqueous solution containing 1 to 3 % by weight of the gelling agent forms a self-supporting, flexible gel on cooling to a temperature of 35 °C or less, preferably 30 °C or less, more preferably 25 °C or less.

The gelling agent gelatin, may be present in the composition in an amount of 6 wt % or less, preferably of 5 wt% or less, most preferably 3 wt% or less. The gelling agent is preferably present in the composition in an amount of from about 1.5 wt% to 2.5 wt%, more preferably about 1.8 to 2.2 wt%, most preferably in an amount of about 2 wt %.

The composition may comprise a mixture of a first gelling agent and a second gelling agent, different from the first gelling agent. The first gelling agent may be as described above. The second gelling agent, which may be the same as the lubricating agent may be hydroxypropylmethylcellulose.

As mentioned above, the gel is a mixture which includes a lubricating agent to improve the gel's lubricating effect in the mouth and throat in use. The lubricating agent is preferably itself able to form a gel with water. The lubricating agent HPMC, may be present in the composition in an amount of 3 wt% or less, more preferably 2 wt% or less, most preferably 1 wt% or less. Preferably, the lubricating agent HPMC, is present in the composition in an amount of from 0.1 wt % or more, preferably 0.2 wt% or more. Preferably, the lubricating agent HPMC, is present in the composition in an amount of from about 0.2 to 0.6 wt%, more preferably about 0.3 to about 0.5 wt %, more preferably about 0.35 wt% to about 0.45 wt%, most preferably about 4 wt%.

Preferably, the lubricating agent comprises (for example consists essentially of, e.g. consists of) a synthetic cellulose derivative having the ability to influence the lubricating properties of the gel on contact with mouth or throat fluids in use. Such cellulose derivatives include, for example, hydrophilic cellulose drivatives with water-keeping actions such as carboxymethylcellulose, methylcellulose, hydroxypropylmethylcellulose (HPMC), and combinations thereof. HPMC is known in the food industry as a gelling agent. It has been found to be surprisingly effective in acting as a lubricating agent in gels formed from other gelling agents. The gelling agent is preferably present in the composition in an amount of 0.1 to 5 wt %, preferably 0.2 to 1.5 wt%, most preferably about 0.3 to 0.7 wt %.

Preferably, the composition comprises (more preferably consists essentially of, for example consists of) gelatin and HPMC. The combination has been found to be particularly effective, since such compositions have sufficient structural integrity for suitable handling, can successfully assist in the swallowing of an edible object that would otherwise be difficult to swallow. Further improvements can be found in altering the relative ratios of the gelatin and HPMC. Certain compositions containing both components have been found to be surprisingly more rigid than one would expect from the components alone. It is believed there is a rheological synergy between the two components. Furthermore, as well as providing more rigidity in some circumstances, the HPMC acts as a surprisingly effective lubricant.

Preferably, the edible gel composition comprises gelatin and hydroxypropylmethylcellulose (HPMC) in w/w ratio of about 1:1 to about 10:1. More preferably, the edible gel composition comprises gelatin and hydroxypropylmethylcellulose (HPMC) in w/w ratio of about 6:1 to about 3:1, optionally from about 5:1 to about 3:1, optionally from about 5.5:1 to about 3.5:1. In an embodiment, the edible gel composition preferably comprises gelatin and hydroxypropylmethylcellulose (HPMC) in w/w ratio of about 4:1. In an embodiment, the edible gel composition preferably comprises gelatin and hydroxypropylmethylcellulose (HPMC) in w/w ratio of from about 6:1 to about 4:1, optionally of from about 5.5:1 to 4.5:1, most preferably of about 5:1.

The edible gel composition may consist essentially of or consist of gelatin, hydroxypropylmethylcellulose (HPMC) and water. A composition that "consists essentially of" these three components includes, but is not limited to, a composition that contains 5 w/w% or less of other components, preferably 2 w/w% or less of other components, most preferably 1w/w% or less of other components. No other components are detectable in a composition that consists of gelatin, hydroxypropylmethylcellulose (HPMC) and water.

In a preferred embodiment, while in contact together, the gel does not react with the medicines or their coatings and/or reduces tackiness when entering the mouth to ease swallowing. The gel preferably does not contain any sugar, making it suitable for those patients on a controlled diet. The gel preferably does not contain an inorganic salt, for example sodium chloride.

The edible gel composition may further comprise one or more additives, for example one or more flavourings and/or one or more preservatives. Such flavourings and preservatives may be selected from any that are suitable for oral administration to a subject, such as a human or animal. The one or more preservatives preferably mitigate or prevent microbial growth, for example bacterial and/or fungal growth.

Preferably the composition or bolus will have sufficient structural integrity that it will not disintegrate or shed particles into the esophagus on ingestion. In this way, the danger of aspiration of particles into the lungs is minimised and the gel will also serve to contain any particles that might be shed from the dosage form contained in it.

The avoidance of aspiration of particles into the lungs via the trachea and larynx is an important advantage of the present invention. Several studies on dysphagia have related the effects of bolus consistency with the physiology of the deglutition process. It was observed that liquid boluses were easily propelled through the oral cavity, even in the presence of impaired deglutition function, but result in a higher risk of laryngeal tracheal aspiration due to premature pharyngeal spillage. Aspiration of unsterile medicines or foodstuffs into the lungs increases the change of both upper and lower respiratory tract infections e.g, pneumonia.

Additionally patients or carers can resort to crushing tablets or opening capsules to facilitate administration of the medicine. This action can prove dangerous as in releasing all the contents of a slow release drug at one time resulting in higher than optimum drug levels. Moreover, some coated tablets contain drugs which are inactivated by the stomach pH or are an irritant in the stomach, again potentially causing adverse effects. Despite the evidence, in the case of swallowing difficulties, crushing tablets by nurses and carers during administration of medicines is still a common *modus operandi* (Mistry et al., 1995, Belknap et al., 1997, Tissot et al., 1999, Wright, 2002, Kirkevold and Engedal, 2005).

The use of the gel according to the present invention enables solid dosage forms to be administered without crushing, without damaging or destroying the enteric or delayed-release coatings of the solid forms and without having to resort to liquid formulations which are typically more expensive and lack the enteric or delayed-release coatings of solid forms. The risk of aspiration of particles is also significantly reduced by the present invention, both by the containment of stray particles by the gel and the avoidance of crushing of the dosage form.

Increasing bolus viscosity causes several modifications of the swallowing performance and facilitates the swallow triggering since more time is given for the reflex (Logemann, 1983). Studies using manometry or videofluoroscopy, showed that oropharyngeal muscle activity increases with increments of bolus viscosity (Cooke et al., 1989, Lazarus et al., 1993). In the presence of increased bolus viscosity the tongue base-pharyngeal wall contraction increases, the duration of peristaltic waves is prolonged, the velocity of oesophageal peristaltic waves is slowed and the upper oesophageal segment opening is prolonged (Kuhlemeier et al., 2001). Liquid boluses should be easily propelled through the oral cavity even in the presence of an impaired swallowing reflex; however, higher risks of laryngeal tracheal aspiration, due to premature pharyngeal spillage may occur due to loss of bolus control. If bolus consistency is increased its control by the tongue is easier (Miller and Watkin, 1996).

Therefore, the importance of increased viscosity for safe swallows for dysphagic patients and the need to avoid thin boluses that spill into the lungs is clear (Deward and Joyce, 2006). Additionally too viscous boluses require greater energies to be swallowed and should also be avoided (Miller and Watkin, 1996).

The gel for use in the present invention is preferably selected to have a relatively high viscosity so that the transit time in the throat is slowed relative to a conventional dosage form, giving more time for airways to close and give protection against undesired aspiration of particles. Simultaneously, the swelling of the gel on contact with the fluids of the mouth and throat increases the lubricity and decreases the tackiness of the gel so that the adherence onto the internal throat surfaces is reduced sufficiently for the bolus to be swallowed even by a patient with moderately or severely impaired swallowing function.

Preferably, the recess is a slit or aperture, which may be formed when the gel composition is formed, e.g. from a liquid pre-gel mixture, by using a mould having an appropriate protrusion, such than when the gel is removed from the mould, a recess is present in the surface of the composition. Alternatively, the recess may be formed by solidifying the pre-gel in a mould, and then subsequently cutting a recess, e.g. a slit or aperture, in the composition. The skilled person could use any suitable instrument to cut the recess, such as a sharpened blade. The recess may be made by hand or by mechanical means, e.g. in an automated device.

The dosage form may comprise any solid or semi-solid pharmaceutical dosage form, which may comprise a pharmaceutically acceptable excipient and an active component. The dosage form may be in any form known to the skilled person, such as a powder, a tablet, pill or capsule. The dosage form may be a unit dosage form. A "unit dosage form" includes, but is not limited to, physically discrete units suitable as unitary dosages for human subjects and other mammals, each unit containing a predetermined quantity of active material calculated to produce a desired therapeutic effect, in association with a suitable pharmaceutical excipient (e.g., a tablet, capsule, ampoule). In an embodiment, the dosage form may comprise, consist essentially of or consist of a pharmaceutically active component. It the dosage form consists essentially of a pharmaceutically active component, this includes a dosage form that comprises a pharmaceutically active component and preferably less than 5 wt % of other components, more preferably less than 2 wt % of other components. The dosage form may be associated with the edible composition immediately prior to administration, for example 30 minutes or less, preferably 15 minutes or less, more preferably 5 minutes or less, before the edible composition is consumed.

In an embodiment, the present disclosure provides a dosage form comprising, consisting essentially of or consisting or a composition of the present invention and a pharmaceutically active component. The pharmaceutically active component may be covered by the composition of the present invention. The pharmaceutically active component may be dispersed throughout the composition of the present invention. In an embodiment, the pharmaceutically active component, optionally in its pure form, may be inserted into the composition immediately prior to administration of the composition orally to a subject.

The edible composition is preferably prepared using one or more methods described below.

The gel composition may be of any suitable size and shape that allows it to be swallowed. The gel composition may be termed an administration unit. Preferably, the administration unit has a minimum diameter of about 0.5 cm. Preferably, the administration unit has a maximum diameter of about 1.5 cm. The recess may be a cut or aperture with a length of about 0.3 to 1 cm as measured across the surface of the composition. The recess or aperture may have a depth of about 0.2 to 0.7 cm. It may have a width of 0 cm (i.e. a cut in which the walls meet and require movement apart to insert a dosage unit) or more, up to about 0.3 cm. The administration unit may be of approximately hemispherical shape, i.e. with a shape with a flat base and a rounded section. The recess is preferably formed in the flat base. The administration unit may have an elongated shape. The shape may be a cylindrical shape, and a recess may optionally be present on the flat end of the cylinder or on the curved side of the cylinder. The administration unit may have a cylindrical part and one or both ends of the unit may taper inwards, e.g. in the shape of a bullet. The diameter of the cylinder may be greater or less than the length of the cylinder (measured along its axis). The largest dimension of the administration unit may be 1.5 cm or less. If in the form of a cylinder, the diameter of the cylinder may be of from 0.5 to 1 cm, preferably about 0.75 cm. The recess may be an aperture with a shape confirming to the shape of a rounded tablet or a capsule. The length of the cylinder may be 1.5 cm or less; the length is preferably 0.5 cm or more.

The present invention provides a method for preparing an edible gel composition for oral administration to a subject with difficulty in swallowing, the method comprising
providing a liquid pre-gel composition for forming a gel;
and then either:
(i) solidifying the pre-gel composition in a mould such that a recess for acceptance of a dosage form is formed in the solidified edible gel composition, or
(ii) solidifying the pre-gel composition in a mould and forking a recess in the solidified gel, e.g. by cutting a recess in the gel.

The present invention provides a method for preparing an edible gel composition for oral administration to a subject with difficulty in swallowing, the method comprising
providing a liquid pre-gel composition comprising
a gelling agent,
water, and
a lubricating agent; and
solidifying the pre-gel composition in a mould. Optionally, a recess may be formed in the gel composition by either:
(i) solidifying the pre-gel composition in a mould such that a recess for acceptance of a dosage form is formed in the solidified edible gel composition, or
(ii) solidifying the pre-gel composition in a mould and forming a recess in the solidified gel, e.g. by cutting a recess in the gel.

The gelling agent may be as described herein. The lubricating agent may be as described herein. The method may comprise forming an aqueous liquid pregel mixture of the gelling agent and combining this with the lubricating agent. If the lubricating agent forms a gel with water, an aqueous pre-gel mixture comprising both the gelling agent and the lubricating agent may be formed, and then allowed to solidify. The method may involve forming an aqueous liquid pregel mixture of the gelling agent and an aqueous liquid pregel mixture of the lubricating agent, and combining the two pre-gel mixtures, and then allowing them to solidify.

The present invention further provides a method of administering a dosage form to a subject with difficulty in swallowing, comprising
providing a composition of the present invention having a dosage form associated with the composition;
administering the composition orally to the subject.

The present invention further provides a method of treating a subject with difficulty in swallowing and optionally an associated disorder, the method comprising administering orally a composition of the present invention having a dosage form associated with the composition, the dosage form being for the treatment of the difficulty in swallowing and/or the associated disorder.

The dosage form may be associated with the composition. "Associated with" includes, but is not limited to, in contact with, such that the ease with which the dosage form is swallowed is improved as compared to the ease with which the dosage form is swallowed without the presence of the composition. The dosage form may be partially or fully coated with the composition of the present disclosure. The dosage form may be present in the recess of the composition of the present disclosure. The dosage form may be fully or partially encapsulated by the composition of the present disclosure in the recess Preferably the composition of the present invention overlies at least part of the dosage form. Preferably, the thickness of the gel composition overlying the dosage form is in the range of about 0.2 mm to 5 mm, preferably I mm to 3mm.

The present invention may be for use in the treatment of a subject with difficulty in swallowing and/or another disorder, which may be a disorder associated with the difficulty swallowing. The present invention further provides the use of a composition of the present invention in the manufacture of a medicament for the treatment of a subject with difficulty in swallowing and/or another disorder, which may be a disorder associated with the difficulty swallowing. Preferably, a dosage form is associated with the composition, and the dosage form is for the treatment of difficulty in swallowing and/or another disorder. It may be an associated disorder, i.e. a disorder associated with and may be causing the difficulty in swallowing.

The associated disorder may be selected from blunt throat injury, surgery-caused impairment, stroke, multiple sclerosis, Asperger syndrome, esophageal cancer, laryngeal cancer, chagus disease, celiac, cystic fibrosis, Huntington's disease, Niemann-Pick disease, neurological conditions such as amyotrophic lateral sclerosis, Alzheimer's and Parkinson's disease, obesity, Riley- Day syndrome, high cholesterol, corn allergies and corn sensitivities, sclerodenna, and diabetes.

The present invention further provides a package containing an edible composition of the present invention. Preferably, at least part of the edible composition is exposed, and a recess is present on the exposed part of the edible composition. The package may comprise a base containing a gel composition of the present invention and a removable cover. The recess in the gel may be exposed on removal of the cover.

The package may comprise:
(i) a base having one or more indentations containing a gel composition of the present invention, the composition having a recess therein for the insertion of a dosage form; and
(ii) a removable cover, such that when the cover is removed, the recess is exposed to allow insertion of a dosage form into the recess.

The base of the package is preferably deformable to allow the ejection of the gel composition from the base, optionally after a dosage form has been inserted into the recess. The base preferably comprises a plastic. Suitable plastics are known to those skilled in the art. The removable cover preferably comprises a metal foil. Suitable metal foils are known to those skilled in the art. The package may be in the form of a "blister pack" known to those skilled in the art. The composition of the present invention may be formed in the package from the pre-gel; the package may act as a mould in the formation process.

The present disclosure further provides an apparatus for testing the ease with which a composition can be swallowed comprising:
an analyser for measuring the resistance of a composition to shear, the analyser comprising a probe that can be moved downwards at a constant speed for a determined distance, and
a substantially vertical hollow tube with an upper entrance, with the probe and the tube arranged such that the composition can placed at the upper entrance to the tube and moved vertically downwards within the tube such that it pushes the composition though the tube at a constant for the determined distance, and the resistance to shear being measured over the distance by the analyser. The probe is preferably of the same cross-sectional shape as the tube, e.g. cylindrical, with a diameter slightly less (e.g. 0.1 to 2 mm less) than the internal diameter of the tube.

The tube may be a cylindrical tube, preferably having a diameter of from 10 to 30 mm, preferably about 15 to 25 mm. The distance moved by the probe is preferably 10 to 30 mm, preferably about 20 mm. The speed is preferably 10 to 30 mm/s, preferably 15 to 25 mm/s preferably about 20 mm/s. The tube preferably has an upper section that tapers outwardly, preferably in a cone shape. The tube preferably comprises plastic, preferably poly(methylmethacrylate), otherwise termed Perspex or acrylic. Any suitable plastic may be used. The analyser may have any one or more of the features of the Texture analyser described in the Examples. The tube is preferably of from 30 to 60 mm in length, preferably 40 to 60 mm in length. The length of the tube is longer than the predetermined distance. The tube preferably includes one or more apertures to allow air flow out of the tube while the composition passes down the tube. The apertures are preferably within the base of the tube or on the side wall of the tube, preferably within 2 cm from the base.

The present disclosure further provides a method for testing the ease with which a composition can be swallowed, the method comprising
providing an apparatus of the present invention,
placing the composition at the upper entrance to the tube, moving the composition down the tube with the probe at a constant speed and for a determined distance, and measuring the resistance to shear over the distance.

The composition is preferably of a shape and diameter such that it can rest on the upper entrance to the tube in the absence of any pressure from the probe, but can be moved through the tube when force is applied by the probe in the downwards direction. The composition is preferably cylindrical in shape. The composition, preferably in cylindrical form, preferably has a maximum diameter greater than, preferably 0.2 to 20 mm greater, than the internal diameter of the tube, preferably 0.5 to 15 mm greater than the internal diameter of the tube, preferably about 10 mm greater than the internal diameter of the tube. The composition, preferably in cylindrical form preferably has a height of 5 to 15 mm, preferably about 10 to 14 mm, more preferably about 12 mm.

In a modification, the "dosage form" in any of the aspects of the invention may instead be read as referring to any solid, ingestible object. Such solid, ingestible objects include, but are not limited to, a solid medicament, a solid food item, a solid snack or sweet item, a solid food supplement form, or the like. Solid medicaments can, for example, include tablets and capsules.

### Brief Description of the Drawings

The present disclosure will now be illustrated with reference to the non-limiting Examples and the accompanying figures, in which:
Figure 1 shows the Perspex device used in the simulated swallowing test, on the instrument's platform, with the 20 mm diameter cylindrical probe above;
Figure 2 shows the Texture Analyser used in the simulated swallowing test, the instrument fitted with the Swallowing Model device of Figure 1: 5Kg load cell (left side) and connected personal computer (PC) (right side);
Figure 3 shows the force required to move (push) a composition of the present invention and a comparative composition through the tube used in the simulated in vitro swallowing test under both wet and dry conditions (n=7); above each of "wet" and "dry", the left-hand bar represents the mixture Gelatin 2% w/w+HPMC 0.5% w/w and the right-hand bar represents pure Gelatin gel 2.5% w/w;
Figure 4 shows the percentage increase in weight of a composition of the present invention and a comparative composition when contacted with water over a period of 60 minutes; the effect, on the weight of the mixture Gelatin 2% w/w+HPMC 0.5% w/w (■) and pure Gelatin gel 2.5% w/w ( 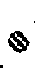 ), of increasing immersion time in the water at 21°C is shown;
Figure 5 shows a schematic human throat vertical cross section during swallowing of a composition or bolus of the present invention (white circles) containing a dosage form (black central dots) and entering from the mouth into the superior part of the throat; the high gel viscosity slows the tablet transit giving more time for airways closure and protection, and the swelling properties of the composition decrease the adherence onto the internal throat surfaces;
Figure 6 shows a schematic vertical section of the human throat during the passage of the composition shown in Figure 5; the risk of aspiration of small tablets or other dosage forms, or of particles that may be shed by the dosage form, is decreased by using the gel in accordance with the invention.
Figure 7 shows the responses to the statement "the swallow was comfortable" for the conditions with or without water under the clinical tests described in Example 4;
Figure 8 shows the responses to the statement "the swallow was comfortable" for the different bolus sizes under the clinical tests described in Example 4;
Figure 9 shows the responses to the statement "the swallow was comfortable" for the hard and soft gels under the clinical tests described in Example 4;
Figure 10 shows the responses to the statement "the swallow was easy" for the conditions with or without water under the clinical tests described in Example 4;
Figure 11 shows the responses to the statement "the swallow was easy" for the different bolus sizes under the clinical tests described in Example 4;
Figure 12 shows the responses to the statement "the swallow was easy" for the hard and soft gels under the clinical tests described in Example 4;
Figure 13 shows the responses to the statement "the gel felt sticky" for the conditions with or without water under the clinical tests described in Example 4;
Figure 14 shows the responses to the statement "the gel felt sticky" for the different bolus sizes under the clinical tests described in Example 4;
Figure 15 shows the responses to the statement "the gel felt sticky" for the hard and soft gels under the clinical tests described in Example 4; and
Figure 16 shows the results of studies into compression of gelatine and HPMC, measuring the force required in grams to compress the gel to a depth of 5mm (experiments carried out at room temperature on gels that had been left to equilibrate for one hour).

### Examples and Detailed Description of the Drawings

### Materials

**Table 1 : Materials used**

| **Material** | **Supplier code** | **Supplier** | **Note** |
|---|---|---|---|
| Gelatin type A | G2500 | Sigma, St. Louis MO | Derived from Porcine skin |
| Hydroxypropyl methylcellulose (HPMC) 2208 | Methocel K4M Premium EP | Colorcon | HPMC meets European Pharmacopoiea requirements; certified Kosher |

### Example 1 - Gel material

**Gel preparation method:** The total amount of water necessary to prepare a certain concentration was shared in two equal volumes: one part was used to prepare pure HPMC and one for pure Gelatin. HPMC (used as received) was first dispersed in 2/3 of the water (at 80-85°C), stirred for 30 minutes and allowed to cool at the room temperature before pouring up to the required volume of water. Gelatin gel was prepared by stirring the solution for 10 minutes at 70°C. The two solutions obtained were then mixed prior to gelification and the gel was allowed to form at room temperature and then stored for 24 hours at 10 ° C.

**Example:** to prepare 100 ml of a gel made of Gelatin 2% w/w and HPMC 0.5 % w/w:
2 g of Gelatin
0.5 g of HPMC
97.5 ml of distilled water at room temperature were used.

The described preparation method was followed. The samples of Example 1 were moulded in cylindrical moulds to form samples having a diameter of 30-31mm and a height of 11-12 mm.

### Example 2 - Alternative gel material

A gel was prepared in accordance with Example 1, except that no HPMC was added to the pre-gel mixture. The gel contained 2.5% w/w of gelatin. This was formed from 2.5g of Gelatin in 97.5ml of water using the same temperature and conditions as Example 1.

The samples of Example 2 were moulded in cylindrical moulds to form samples having a diameter of 30-31mm and a height of 11-12 mm.

### Example 3 - Simulated swallowing test

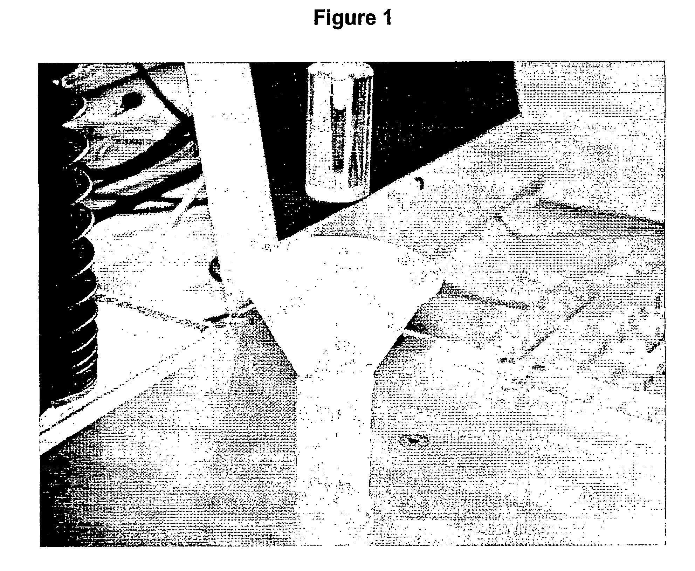

**Details of the cylinder (the model)** The model consists of a plastic device (see Fig. 1) and it is assembled with the instrument Texture Analyzer (model TA-XT*plus*). The model itself consists of two parts: the top part with a conical shape and the lower part is a cylinder of 20 mm internal diameter and 50 mm height, with 4 holes around the bottom (approximately 1 mm diameter each). The whole model is in plastic and is empty. It is designed to be able to hold the sample, and to contain the 20 mm diameter cylindrical probe (standard perspex probe provided by the instrument's manifacturer Stable Micro Systems). During testing, this model is placed on a fixed support, over the platform of the instrument Texture Analyzer and the probe, just above it, is held by the 5 Kg load cell of the instrument and able to push the sample into the model.

Test method The sample is placed on the top of the main structure of the Perspex device and it is subjected to shear by the movement of the probe, controlled by the instrument and pushed into the cylindrical part of the model. The probe head is a close fit in the cylindrical part of the model and the amount of shear applied to the substance, in such an arrangement, can be accurately recorded by the software of the instrument. The probe is programmed to travel at a defined and constant speed, up to a chosen depth (test parameters are reported below), and the sample is subjected to shear, as a result of the action of controlled forces, exerted by the Texture Analyzer instrument. The cylindrical probe, connected with the load cell of the Texture Analyzer, is then placed close to the sample surface, but not in contact. When the test starts, the probe starts to move at a selected speed and goes down to a defined distance. The sample comes in contact with the probe and then it is subjected to controlled shear.

**Measurements:** Values for the resistance of the gel to shear are determined for defined deformations. The amount of shear, applied to the sample, in such an arrangement, is accurately controlled by the Texture Analyzer and recorded. The value of the resistance, of the sample to shear, is given as the Maximum force (expresses in grams), which is necessary to obtain a certain defoliation, at a defined probe speed.

**Table 2 - Test method details**

| | |
|---|---|
| **Mode** | measure force in compression |
| **Option** | return to start |
| **Pre-Test speed** | 3 mm/s |
| **Test-speed** | 20 mm/s |
| **Post-Test speed** | 20 mm/s |
| **Distance (depth)** | 20 mm |
| **Trigger type** | Auto - 10g |
| **Tare mode** | Auto |
| **data acquisition rate** | 200 pps |
| **Accessory** | 20 mm diameter cylinder Perspex probe using 5 Kg load cell |

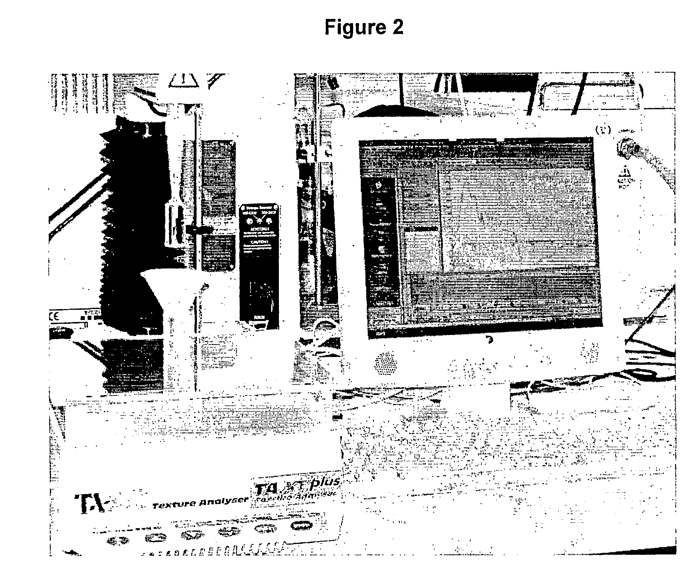

**Details of the testing machine** The instrument Texture Analyzer (model: TA.XT*Plus* - see Fig. 2) and the software Texture Expert Exponent Software are both designed by Stable Micro Systems, Ltd in Godalming, Surrey, UK.

**Table 3 - Instrument Texture Analyzer (model TA-XTplus) specifications**

| | **5 Kilo Load Cell** |
|---|---|
| **Max Force** | +/- 5Kg |
| **Force Sensitivity** | 0.1 g |
| **Load Cells** | Directly interchangeable by the user. All loadcells store their unique calibration and identification information in 'onboard' non-volatile memory |
| **Speed Range** | 0.01 - 40mm-second ( 20mm/second at 50kg max. range) |
| **Speed Accuracy** | Better than 0.1% |
| **Position Range Setting** | 0.001-295mm (or 545 mm with Extended Range TA.XTPlus) |
| **Range Resolution** | 0.001 mm |
| **Operating Modes** | Measurement of force and distance in tension or compression. |
| **Statistics** | Mean and Standard Deviation (in the instrument) and virtually any calculation via the Texture Expert or Texture Expert Exceed software programs. |
| **Data Aquisition Rate** | Up to 500 points per second (pps) for cach data channel. |
| **PC Interface** | Interface to PC through a standard RS232 serial port at 115200 BAUD. |
| **Atmospheric** | Laboratory conditions. Dust and splash resistant |
| **Operating Temperature** | 0 - 40° C (32-104° F) |
| **Overall Dimensions** | 665 mm (26.2") tall x 440 mm (17.3") deep x 280 mm (11") wide |
| **Weight** | 16.2 kg |
| **Power Supply** | Universal mains input voltage (85-264 volts AC at 47-63 Hz) |

(The instrument specifications shown above were publicly available on the website http://www.texturetechnologies.com/ on 10^{th} Oct 2007)

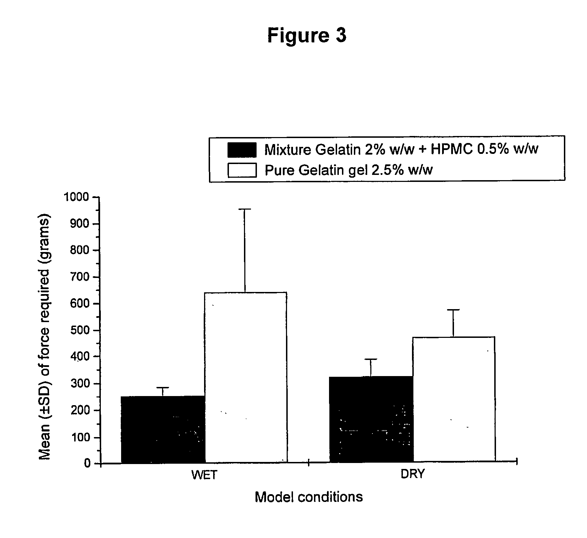

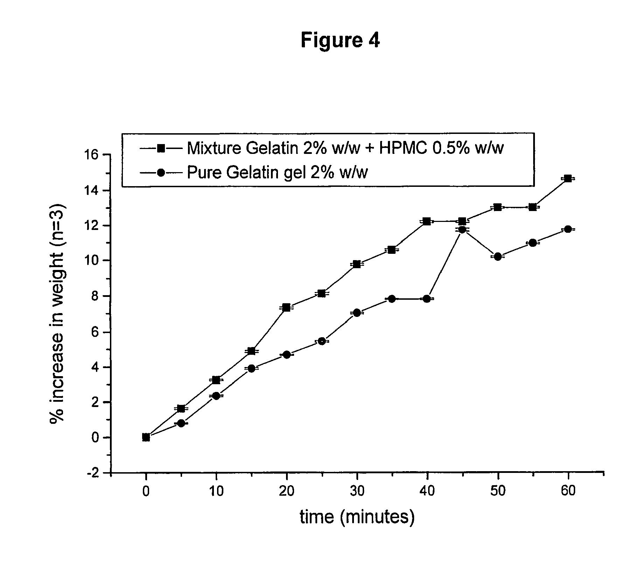

Results The mean (sd) force required to push the a composition of Example 1 under wet conditions, simulating the throat milieu, was 252.23±31.5 grams, compared to 320.69±63.8 grams under dry conditions (p<0.05, Independent samples t-test). The mean (sd) force required in the formulation without the additive in wet and dry conditions respectively was 639.9 grams (311.8) & 468.23 grams (101.9) (p=0.154, Independent samples t-test). (see Fig. 3) Moreover, swelling data (see Fig. 4; it should be noted that in Figure 4 "Pure Gelatin Gel 2 % w/w" should be replaced with "Pure Gelatin Gel 2.5 % w/w") reveal that the gel swells on contact with water and the weight increases linearly with time (> 14% within 1 hour) which suggests that the medium penetrates into the gel in the same way during the whole test; whereas, the penetration speed increases with time.

**Conclusions** The innovative *in-vitro* model is simple to operate, providing good reproducibility and accuracy. The model demonstrates that the composition comprising gelatin and HPMC will be swallowed with greater ease than a composition comprising gelatin alone. In addition, the swelling study clearly showed the ability of the formulation to absorb water.

### Example 4 - Investigations into Effects of Gel Consistency and Bolus Size

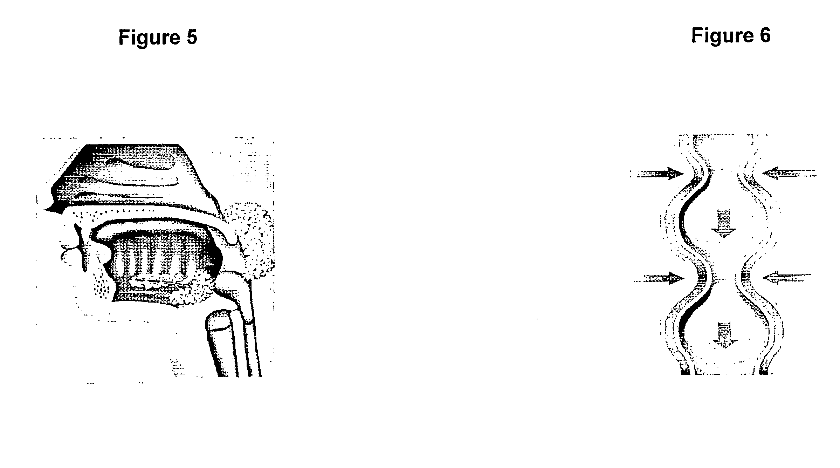

The potential advantages of administering tablets or capsules (shown in Figures 5 and 6) within our gel are:
● increased tablet weight thereby reducing transit time i.e. swallowing time
● marginally increased bolus size thereby making small tablets more manageable
● appropriate viscosity which guarantees that the bolus passage is not too fast, thereby providing time for safe airway closure
● increased slipperiness of tablet or capsule in contact with saliva and therefore reduced need for water to facilitate swallowing
● provision of a barrier between the drug and the oral mucosa thereby preventing local irritation and masking the taste of the medicine.

In order to obtain a formulation suitable for people with swallowing difficulties, two formulation properties were considered of primary relevance during formulation development, namely gel consistency and gel size. Firstly, it was envisaged that the appropriate texture of the final formulation would have been at least a reasonably soft gel, to encourage ease of swallowing but, at the same time would have had a texture such that no residue was left inside the throat during swallowing. In fact, especially in subjects with dysphagia, the risks of aspiration of small particles (or little gel pieces) are very high.

Moreover, it was thought that the ideal gel size would have had a diameter similar to the human throat section, in order to avoid the natural tendency of chewing it when in the mouth. At the same time it was necessary to choose dimensions such that the gel was able to hold a tablet or a capsule within itself. Regarding the most appropriate gel size to be adopted for such a formulation, the literature about dysphagia represented the main source of information. Most frequently, these studies reported data about liquids or viscous boluses such as yogurt (Miquelin et al., 2001) rather than solid boluses. However, other examples exist such as the study conducted by Bardan et al. (2004) who studied the bolus kinematics by using manometry-fluoroscopy during the pharyngeal phase of swallowing with young and elderly subjects while swallowing both 15ml liquids and solid boluses of 20mm diameter. In addition, in a review by Chen (2008) it was reported that a standard pharyngeal cross-section diameter measures around 0.02m and this was used to calculate the Reynolds number for the turbulent flow of a water mouthful. The same pharyngeal dimensions were assumed in a study using a biomagnetic method in order to calculate the pharyngeal bolus flow (Miquelin et al., 2001). Therefore, for further formulation characterisation gels having dimensions close to this value were considered appropriate and thus were adopted.

As a prerequisite to clinical application of this gel, it was considered of relevance to establish its in-vivo performance during swallowing in healthy adults, in order to investigate effectiveness, safety and preferences regarding size and texture of the swallowed gels. Thus a clinical trial with healthy volunteers has been conducted at the Ear, Nose and Throat Department at the Norfolk & Norwich University Hospital (NNUH, Norwich), using the diagnostic technique Fiberoptic Endoscopic Examination of Swallowing (FEES^{®}) (see details below).

Receptors in the mouth respond to different characteristics of the bolus and the tactile feedback determines the correct size and position of the bolus before triggering the swallow (Fillion and Kilcast, 2001). Therefore, in order to increase the acceptability of the formulation by patients with reduced swallowing ability and/or sensory perception, the oral sensory feedback of the novel formulation was also investigated during the clinical trial by using an un-validated questionnaire. The questionnaire was conducted with each participant in order to evaluate the sensory perception of such formulation by using discriminant and descriptive methods. Generally speaking, descriptive methods aim to identify or determine the presence of a particular bolus characteristic; whereas, discrimination testing simply indicates whether the products being tested are perceived to be different (Piggott et al., 1998).

### Aims

The aims of this clinical trial were:
● to visualise the in-vivo performance of the gel.
● to establish the optimum characteristics for such a gel.
● to determine how changing gel strength and size increases the likelihood of gel fracture, separation of tablet of gel affects the rate of swallow.
● to assess the patient's perception of the swallow.

### Method

The trial protocol was developed by the Chief Investigator (CI) in consultation with the research team. This protocol was successfully submitted to the local Ethics and Governance Committees.

### Inclusion criteria

The participation of the supervisors in the clinical trial was discussed within the research team before the trial started in order to avoid any possible risk of bias in the study due to their participation. Firstly, it was of primary relevance that before the clinical trial was conducted, the research team had no preferred characteristics for the final formulation. Therefore, it was believed that there was no risk for bias to be introduced as the supervisors had no preconceived ideas as to what they would prefer. Additionally, in order to guarantee maximum privacy it was decided to arrange two individual examinations so each participant had no chance to be influenced by others' answers. With regard to FEES^{®}, as reported by Dodds (1989), the pharyngeal and oesophageal phases of swallowing are both entirely involuntary and the swallow centre program regulates the peristaltic contraction of pharynx and oesophagus through vagal efferent signals. Hence, based on this evidence, it was established that during the clinical trial there was no possibility to subjectively coordinate or control the swallowing process with voluntary actions.

### Materials

The product tested during the clinical trial was made of two ingredients in distilled water: gelatin which is obtained from animal skin, tendon and cartilage; in particular, the type in use for this formulation is obtained from porcine skin; and hydroxypropyl methylcellulose (HPMC) which is derived from cellulose, the main component of green plants. Both gelatin and HPMC are GRAS products and this means that the Food and Drug Administration (FDA), USA, designated them as "Generally Recognised As Safe" under the conditions of its intended use (Cheeseman and Wallwork, 2002). They are both widely used in the pharmaceutical as well as in the food industries and that guarantees optimal knowledge of their properties; however, their combination is innovative and consequently their properties as a mixture were investigated in this thesis. These two polymers were chosen respectively for fast dissolution rate and high swellability when in contact with fluids; together with their ability to form a soft gel when mixed together over certain concentrations and quickly dissolve at body temperature.

### Hydroxypropyl methylcellulose (HPMC)

Ethers of cellulose, such as HPMC, are water-soluble polymers derived from cellulose, the most abundant polymer in nature. They are used in a wide range of applications as thickeners, binders, film formers and many other applications for food as well as drug applications (National Organic Standards Board Technical Advisory Panel Review, 2002). As food and drug additives they do not add any flavour to the diet, but they improve the textural properties.

### Gelatin (Type A)

Gelatin is a substantially pure protein food ingredient obtained by the thermal denaturation of collagen (Gilsenan and Ross-Murphy, 2001). In the gelatin gel rigidity is obtained once sufficient linking points have been formed to make a three dimensional network and with time further molecules form an ordered structure. It forms thermally reversible gels with water, and the resultant gel shows a melting temperature below body temperature (< 35°C), which gives gelatin products unique organoleptic properties and flavour release. The unique properties characterising gelatin: "melt-in-mouth phenomenon", thermo-reversibility and ability to gel upon cooling allow the aforementioned widespread functionality. Finally, for such formulation gelatin has been selected mainly for three properties: odourless, tasteless and melting temperature at body temperature (Wiley and Sons, 2000).

### Product quality

Moreover, for this clinical trial, which involved human participation, actions were taken in order to guarantee the participants' health and safety through materials bio-compatibility and high quality. Therefore, Certificates of Analysis (COA) and Product Information Sheets from the products' manufacturers were carefully studied and their suitability verified by the CI and the research team.

Gelatin, type A from porcine skin, which is both odourless and tasteless was utilised. One single batch of gelatin (CAS number: 90007-70-8) of this type was used throughout the study, 035K0195 which was supplied by Sigma (UK) and used as received. The product is a light yellow powder with an average molecular weight between 50,000 and 100,000. The following specifications were obtained from its Certificates of Analysis (COA) and Certificate of Origin: appearance - off-white; solubility - slightly hazy faint yellow; protein by biuret - 82%; gel strength (Bloom gelometer) - 292; recommended retest - May 2010; QC release date - May 2005.

Hydroxypropyl methylcellulose (HPMC) used in this study was Methocel type K4M Premium EP, which contains: 22% of Methoxyl and 7.0-12% Hydroxypropyl groups. One single batch of HPMC (type USP 2208) of this type was used throughout the study, SC28012N12, which was supplied by Colorcon (trademark of the Dow Chemical Company) and used as received. The following information was obtained from its product specifications sheet: description - white to slightly off white, fibrous or granular powder; pH (1% solution) 5.5-8.0; identity - meets the requirements of the USP and PhEur; shelf-life - recommended five years if stored in closed containers.

References used for mixtures preparation method:
"The Dow Chemical Company": "Technical Handbook-METHOCEL Cellulose Ethers". Printed in USA, Published September 2002. Form No. 192-01062-0902
Gilsenan, Paula M. and Ross-Murphy, S. B. (2001) "Shear creep of gelatin gels from mammalian and piscine collagens", International Journal of Biological Macromolecules, 29(1), 53-61

### Solvent

One batch of drinking water was used for the preparation of every batch used in the two dates of the clinical trial.

### Sample preparation method

The preparation method adopted consisted of making the two solutions separately, according to scientific literature* at controlled temperature and mixing them in precise proportions before gelification occurred. For this study, the high quality of the samples was ensured namely by the simple standard preparation method developed by the Chief Investigator (CI); optimal hygiene conditions and short storage (maximum 24 hours) at low temperature.

A 50mg Ascorbic acid BP (Vitamin C) by Norbrook (Norbrook Laboratories Limited, Newry, Co. Down, Northern Ireland; circa 8mm diameter and 3mm thickness) has been encapsulated within the gel just before the FEES^{®} examination started.

The batches were prepared 24 hours before each of the two FEES^{®} examinations and stored overnight at controlled temperature. The day of the clinic at the NNUH the samples were transferred at the NNUH within a transportable fridge and stored at controlled temperature until the FEES^{®} examination was performed. The tablet was inserted into the gel using a thin metal spatula to cut the gel from the top and penetrate into the centre. The tablet was pushed inside this slit.

### Standard Operating Procedure (SOP)

A Standard Operating Procedure (SOP) was approved by both the Ethics and the local NHS Governance Committees and strictly followed by the CI during batches preparation in order to ensure GLP. The Sterile Services Department (SSD) at NNUH provided all the sterile containers and gloves (EC marked) used during the samples preparation. All the spatulas and magnetic stirrers used were sterilised at the department, sealed and labelled and used only once. Gloves and coats were worn during the preparation time by the CI and the other person in the room (AM).

In order to guarantee the highest accuracy of the preparation content, weights and volumes were measured by the CI and afterwards checked by a second pharmacist (AM) who was present during the whole samples preparation. Records were accurately taken in batch sheets.

### Sample order

By changing the following three characteristics of the sample swallow a factorial design has been used to create eight different permutations:
● strength of the gel (gelatin 2-3% mixed respectively with HPMC 0.5-0.3%w/w)
● water present during swallow or not (20ml);
● relatively small or large gel size (20mm or 25 mm diameter x I 1mm thickness)

Each participant was expected to swallow a sample set of eight samples during one sitting which were given in random sequence, and another eight samples during a second sitting, i.e. 16 samples each participant. All the 16 gel samples, of any dimension, included a tablet of 50mg Vitamin C. The sequence of the permutations has been randomised prior to commencement of the study. Each permutation has been experienced twice by each participant.

### Randomisation

A factorial design was used which means with three factors there were eight different permutations possible and each permutation was repeated once in each volunteer i.e. 16 swallows for each volunteer. It was designed in order to determine whether the gel was strong enough to hold the tablet and not fracture on swallowing; what size of gel healthy volunteers feel comfortable with; and also whether swallowing the gel with water makes it easier or whether saliva on its own is sufficient.

### Water given during FEES^{®} examination

Based on evidence reported in other studies with healthy and dysphagic patients (Adnerhill et al., 1989, Robbins et al., 1987; Logemann, 1986) for the clinical trial the amount of water given to the participants was 20ml. It was given to the participant in a glass and with half of the samples in a random sequence.

### FEES^{®} procedure

The FEES^{®} procedure adopted during the clinical trial followed a standard FEES^{®} protocol and allowed visualisation of samples' transit within the throat during swallowing. The senior therapist safely inserted the 3-4mm diameter telescope up the nostrils and over the back of the throat while the participant was seated and able to hold the glass of water and swallow the samples given one-by-one on a spoon by the CI. The images were recorded on a video-tape and at the same time shown on a screen and seen by the therapist, the participant and the CI in real time. The whole procedure was carried out by the NNUH senior speech and language therapist. Details of sample number, type and size are reported in Table 4 below.

**Table 4 - Sample characteristics given in a random sequence to each participant**

| **Sample no.** | **Sample size** | **Sample dimensions** | **Sample strength** | **Sample composition** | **Water given or not (20ml)** |
|---|---|---|---|---|---|
| 1 | Small | 20mm diameter X 11mm height | Soft (lower gelatin content) | Gelatin/HPMC 2,0/0.5% w/w | No |
| 2 | Small | 20mm diameter X 11mm height | Soft | Gelatin/HPMC 2.0/0.5% w/w | Yes |
| 3 | Small | 20mm diameter X 11 mm height | Hard (higher gelatin content) | Gelatin/HPMC 3.0/0.3 % w/w | No |
| 4 | Small | 20mm diameter X 11mm height | Hard | Gelatin/HPMC 3.0/0.3% w/w | Yes |
| 5 | Big | 25mm diameter X 11mm height | Soft | Gelatin/HPMC 2/0.5% w/w | No |
| 6 | Big | 25mm diameter X 11mm height | Soft | Gelatin/HPMC 2/0.5% w/w | Yes |
| 7 | Big | 25mm diameter X 11mm height | Hard | Gelatin/HPMC 3.0/0.3% w/w | No |
| 8 | Big | 25mm diameter X 11mm height | Hard | Gelatin/HPMC 3.0/0.3% w/w | Yes |

During each swallow the following has been recorded via FEES^{®}:
● whether the gel was intact when entering and leaving the throat;
● whether the tablet was inside the gel on entering and leaving the throat;
● time in camera, i.e. time to swallow.

### Questionnaire development and implementation

The questionnaire used during the clinical trial was designed by the research team in order to investigate criteria such as formulation comfort, stickiness and ease of swallow, which it was believed would be improved. Each questionnaire included five questions and they were asked to the participant after each sample was swallowed (see Table 5). For three of the five questions asked scales were used and the participant was asked to express his/her views within a five-point Likert scale which included the points 'strongly agree, agree, unsure, disagree, strongly disagree' (Smith, 2002). These kinds of answers were chosen in order to enable the participants to rate the experience, while the last two questions (Q4 and Q5) had "Yes" or "No" answers.

**Table 5 - Questionnaire used during each FEES examination**

| **1) Overall gel and tablet were very easy to swallow** | | | | | |
|---|---|---|---|---|---|
| Strongly agree □ | | Agree □ | Unsure □ | Disagree □ | Strongly disagree □ |

| **2) I was very comfortable with gel size** | | | | | |
|---|---|---|---|---|---|
| Strongly agree □ | | Agree □ | Unsure □ | Disagree □ | Strongly disagree □ |

| **3) The gel felt sticky in my mouth** | | | | | |
|---|---|---|---|---|---|
| Strongly agree, □ | | Agree □ | Unsure □ | Disagree □ | Strongly disagree □ |

| **4) The gel didn't break while in the mouth** | | | | | |
|---|---|---|---|---|---|
| Yes □ | | No □ | | | |

| **5) The tablet separated from the gel in the mouth** | | | | | |
|---|---|---|---|---|---|
| Yes □ | | No □ | | | |

### Results

From the 32 samples administered in 7 instances the formulation fractured and 8 instances the tablet separated from the gel during swallowing. Table 6 provides a summary of the effect of the different conditions on separation and fracture. No definite relationships were identified however there seemed to be a trend for the harder gel to fracture and the similarly the larger and harder gel was more likely to separate from the tablet during swallowing. Binary logistic regression analysis found no variables (water presence, size or hardness) were able to predict whether the gel would fracture or separate.

**Table 6 - Effect of different gel conditions on separation and fracture**

| Condition | n | Fractured | Intact | P* | Separated | Combined | P* |
|---|---|---|---|---|---|---|---|
| With water | 16 | 1 (6.25) | 13 (93.75) | 0.333 | 1 (6.25) | 13 (93.75) | 0.333 |
| Without water | 16 | 4 (25.0) | 12 (75.0) | | 4 (25.0) | 12 (75.0) | |
| Small gel | 16 | 2 (12.5) | 14 (87.5) | 1.000 | 1 (6.25) | 13 (93.75) | 0.220 |
| Large gel | 16 | 3 (18.8) | 13 (82.2) | | 4 (25.0) | 12 (75.0) | |
| Soft gel | 16 | 2 (12.5) | 14 (87.5) | 1.000 | 2 (12.5) | 14 (87.5) | 1.000 |
| Hard gel | 16 | 3 (18.8) | 13 (81.2) | | 3 (18.8) | 13 (81.2) | |

| | | | | | | | |
|---|---|---|---|---|---|---|---|
| * Fisher's Exact | | | | | | | |

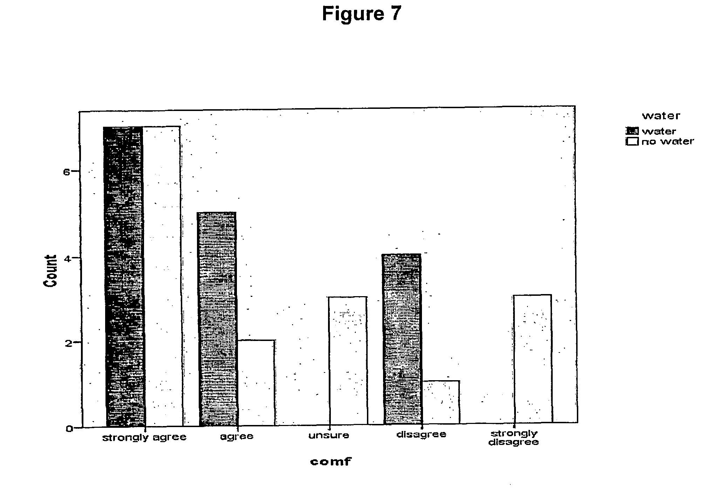

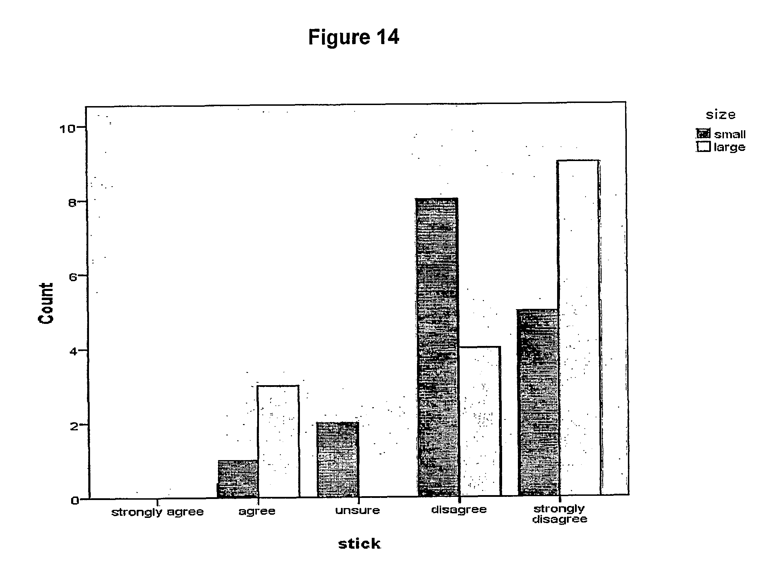

Figures 7-15 provide a summary of views on ease of swallowing, comfort and stickiness under different conditions. It can be see that the presence of water did not seem to be related to comfort or case, whereas there is a preference for the smaller softer gel. Size did not seem to be related to stickiness and again the softer gel was felt to be left sticky.

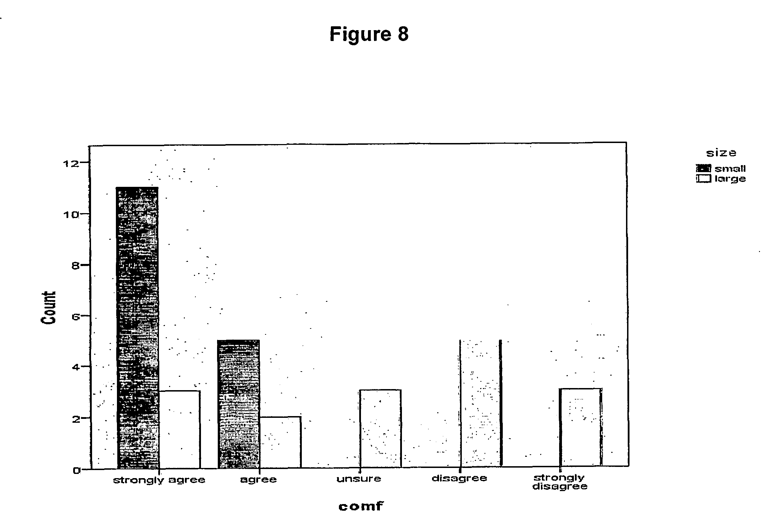

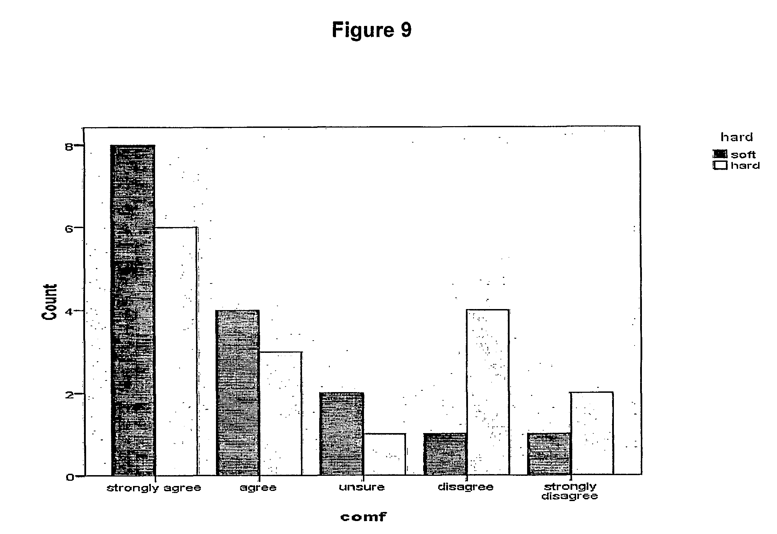

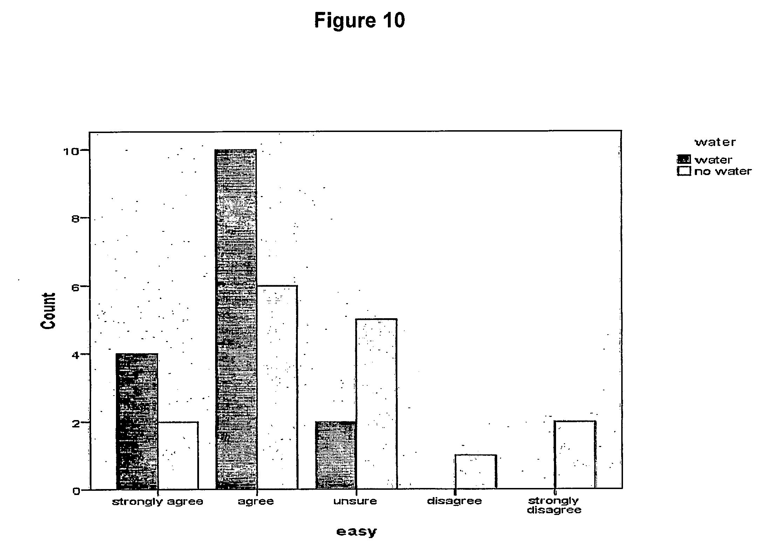

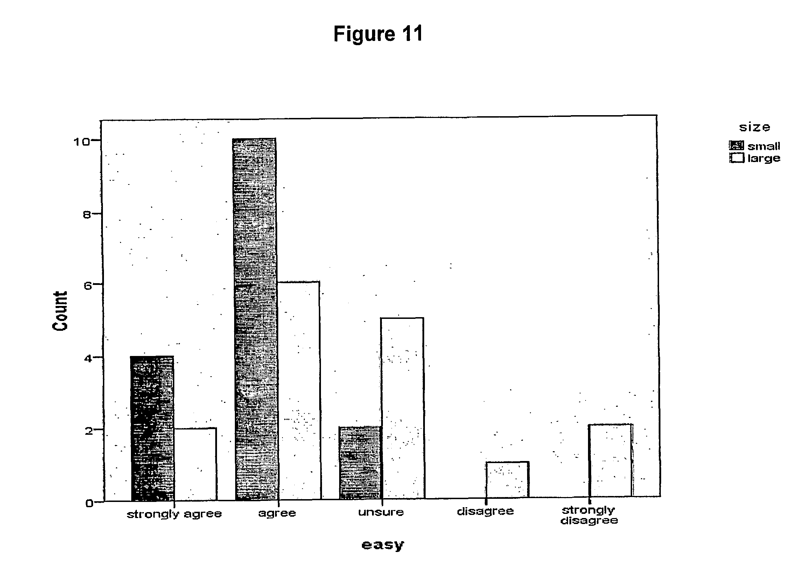

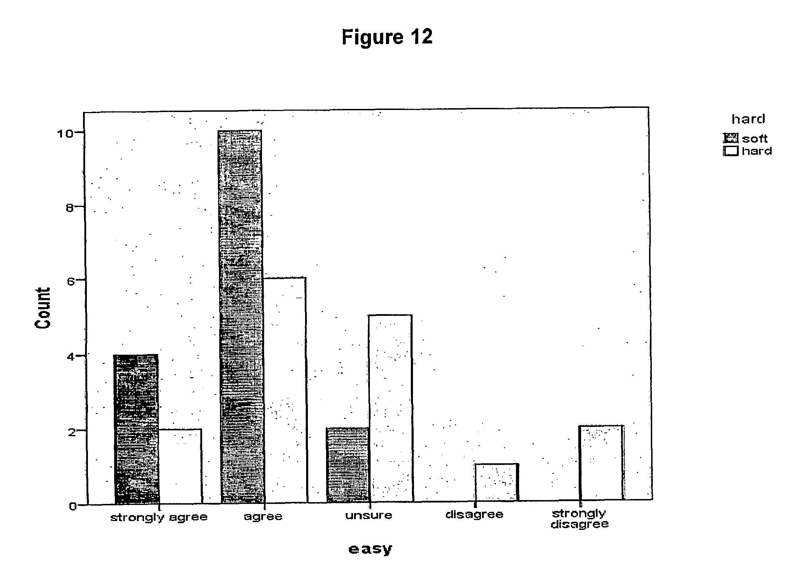

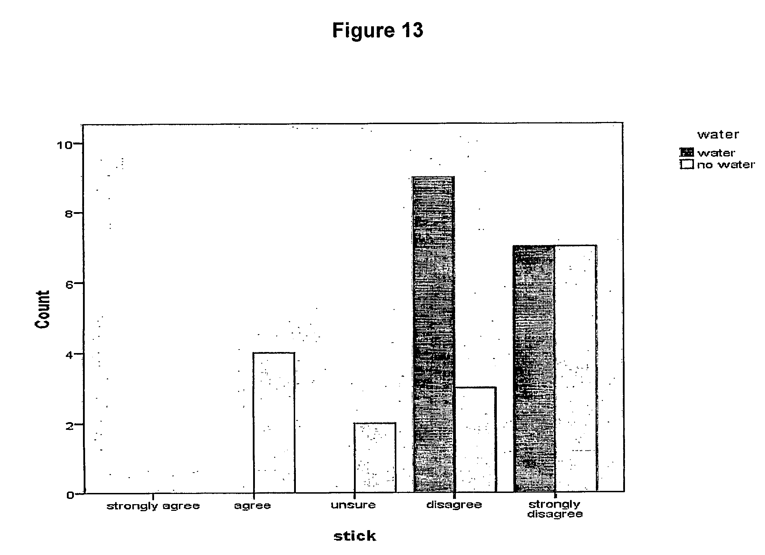

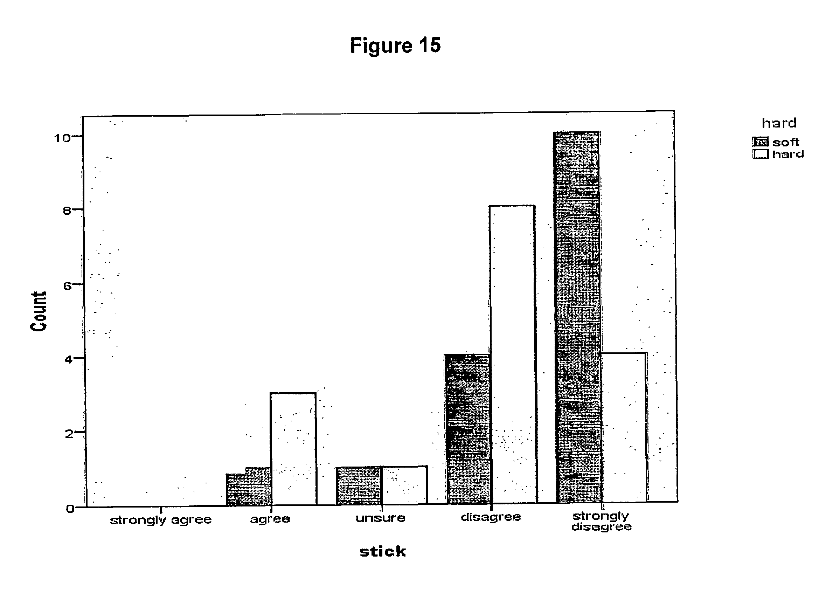

Regression analysis with backward elimination found that size and hardness were the only factors significantly related to comfort (p<0.001 & p=0.097, respectively) with the smaller size causing respondents to move two points up the likert scale on average and the harder gel causing respondents to move half a point down the likert scale on average. Size (Figures 8, 11), the presence of water (Figures 7, 10) and hardness (Figures 9, 12) were all found to be significantly related to case of swallowing, P=0.006 (for all three). Reducing the size of the gel, reducing the hardness and adding in water to the swallow all independently increased case of swallowing by 0.8 of a point on the likert scale. The presence of water (Figure 13) and hardness (Figure 15) were found to be significantly related to the perceived stickiness of the gel, p=0.068 & 0.068 respectively. Moving from the harder to the softer gel and from the absence to presence of water improved responses in each case by two thirds of a point.

### Discussion

The smaller and softer the gel, the more easy and comfortable the swallow. Although the presence of water was not related to the comfort or ease of swallow it was related to perceived stickiness. This may provide evidence for ensuring that the gel should not be used in patients without xerostemia i.e. a lack of saliva, unless water can be given at the same time.

This trial was performed in healthy volunteers and was designed to determine ideal gel characteristics. The evidence supports the use of the gel in aiding tablet administration and demonstrates that the gel as so far developed has potential for this role.

### References

Adnerhill I., Ekberg O., Groher M. (1989) 'Determining Normal Bolus size for thin liquids' Dysphagia 4: 1-3
Bardan E., Kern M., Ardnornfer R., Hofmann C. and Shaker R. (2004) 'Effect of aging on bolus kinematics during the pharyngeal phase of swallowing' Am. J. Physiol. Gastrointest Liver Physiol 290: G458-G465
Chen J. (2008) 'Food oral processing-A review' Food Hydrocolloids doi: 10. 1016/j.foodhyd.2007.11.013
Cheeseman M. and Wallwork J., (2002) 'FDA's Office of Food Additive Safety' Food Safety Magazine by the Target Group, Dec. 2002-Jan 2003
Chisaka H., Matsushima Y., Wada F., Saeki S. (2006) 'Dynamics of capsule swallowing by ealthy young men and capsule transit time from the mouth to the stomach' Dysphagia 21 (4): 275-279
Cook I., Doods W., Dantas R., Kern M., Massey B., Shaker R., Hogan (1989) 'Timing of videofluoroscopic. manometric events and bolus transit during oral and pharyngeal phases of swallowing' Dysphasia 4: 8-15
DeMatteo C., Matovich D. and Hjarterson A. (2005) 'Comparison of clinical and videofluoroscopic evaluation of children with swallowing difficulties' Developmental Medicine and Child Neurology 47:149-157
Deward R. and Joyce M. (2006) 'Time-Dependent Rheology of Starch Thickeners and the Clinical Implications for Dysphagia Therapy' Dysphagia 21 (4): 264-269
Dodds Wylie (1989) 'The physiology of swallowing' Dysphagia 3:171-178
Dodds W., Stewart E., Logemann J. (1989) 'Physiology and radiology of the normal oral and pharyngeal phases of swallowing' AJR 154:953-963
Fillion L. and Kilcast D. (2001) 'Food texture and eating difficulties in the elderly' Food Industry Journal 4 (1): 27-33
Fisher R., Malmud L., Applegate G., Rock E., Lorber S. (1982) 'Effects of bolus composition on oesophageal transit: concise communication' J. Nucl. Med. 23 (10): 878-882
Gilsenan Paula M. and Ross-Murphy Simon B. (2001) 'Shear creep of gelatin gels from mammalian and piscine collagens' International Journal of Biological Macromolecules 29: 53-61
Hey H., Jorgensen F., Sorensen K., Hasselbach H., Wamberg T. (1982) 'Oesophageal of six commonly used tablets and capsules' BMJ 285: 1717-1719
Kikendall J.W., Friedman A.C., Oyewole M.A., Fleischer D., Johnson L.F. (1983) 'Pill-Induced oesophageal injury- Case reports and review of the medical literature' Digestive disease and sciences 28 (2): 174-182
Kuhlemeier J., Palmer J., Rosemberg D. (2001) 'Effect of Liquid Bolus Consistency and Delivery Method on Aspiration and Phayngeal Retention in Dysphagia Patients' Dysphagia 16 (2): 119-122
Langmore S. (2001) 'Endoscopic evaluation and treatment of swallowing disorders' Ed. Thieme, New York, Stuttgart
Langmore S.E. (2003) 'Evaluation of oropharyngeal dysphagia: which diagnostic tool is superior? ' Current opinion in otolaryngology & head and neck surgery 11: 485-9
Lazarus L., Logemann J., Rademarker A., Kahrilas P., Pajak T., Lazar R., Halper A., (1993) 'Effects of bolus volume, viscosity, and repeated swallows in nonstroke subjects and stroke patients' Archives of physical medicine and rehabilitation 74 (10) 1066-70
Martin-Harris B., Logemann J. A., McMahon S., Schleicher M. and Sandidge J. (2000) 'Clinical utility of the Modified Barium Swallow' Dysphagia 15: 136-141
Miller J. L. and Watkin L. (1996) 'The influence of bolus volume and viscosity on anterior lingual force during the oral stage of swallowing' Dysphagia 11: 117-24
Miquelin C., Braga F., Dantas R., Oliveira R. and Baffa O. (2001) Pharyngeal clearance and pharyngeal transit time determined by a biomagnetic methos in normal humans' Dysphagia 16:308-312
National Organic Standards Board Technical Advisory Panel Review (August 26, 2002) 'Hydroxypropyl Methylcellulose' Compiled by Organic Materials Review Institute for the USDA National Organic Program
Ott D. J. and Pikna L. A. (1993) 'Clinical and Videofluoroscopic evaluation of swallowing disorders' AJR 161: 507-513
Piggott J., Simpson S., Williams S. (1998) 'Sensory analysis' Int J. Food Sc. and Techn. 33:7-18
Robbins A., Suffit R., Rosenbek J., Levine R., Hyland J. (1987) "'A modification of a modified barizum swallow'Dysphagia 2: 83-86
Singh and Hamdy (2006) 'Dysphagia in stroke patients' PMJ 82:383-391
Smith F. (2002) 'Research methods in Pharmacy Practise' Pharmaceutical Press, London Chicago
Wiley John and Sons (2000) Cole, CGB. Gelatin. Frederick J. Francis, Editor. Encyclopedia of Food Science and Technology, 2nd edition. 4 Vols. New York: 1183-1188.

### Dysphagia assessment by FEES^{z}

To identify the presence of dysphagia, assess the origin of the symptoms and determine the risk of silent aspiration, several diagnostic laboratory evaluations of swallowing can be used by therapists to treat patients with dysphagia. Patients with difficulties in swallowing undergo clinical evaluation which is conventionally conducted by a Speech and Language Therapist (SLT) testing oral motor functions of the patient using liquid, solid or semi-solid materials, depending on the degree of disability. Low-viscosity liquids, such as water, are used first to better define the reason for aspiration and are more easily cleared by the patient. An important limitation of the clinical bedside examination is that the presence and severity of aspiration is not easily determined (Ott and Pikna, 1993). Clinical evaluation is necessary for determining comprehensive feeding strategies but it is frequently unable to predict or even observe penetration or aspiration phenomena and it can not be used to assess the pharyngeal and oesophageal stages of swallowing (DeMatteo et al., 2005).

Fibreoptic endoscopic examination of swallowing (FEES^{®}) is as a diagnostic tool to assess aspiration of foods and liquids, as well as any abnormality during oral feeding (Langmore, 2001). During a standard FEES^{®} examination only one Speech-Language Pathologist (SLP), who is the endoscopist and another person who assists and feeds the patient are necessary. The person under examination is seated in an upright position or in postures, typical of those in which he/she normally eats and the endoscope is passed through the nose into the oropharynx to a point posterior to the epiglottis, where laryngeal structures can be clearly visualised (Langmore, 2001). The patient is asked to swallow different boluses and the instrument video records its passages.

This technique is able to show if the swallowing problem is in relation to inadequate bolus propulsion, bolus clearing, ineffective tongue propulsion, laryngeal elevation, or hyoid elevation etc. In other cases, it is possible to understand if the problem is in relation to bolus timing propulsion and airway protection, or timely initiation of the two events (Langmore, 2001).

### Example 5 - Comparative Tests With Different Gel Materials

### Materials and Methods

Gelatine, made from porcine skin was obtained from Sigma, Steinham, Germany and HPMC supplied as Methocel K4M was obtained from Colorcon (for the Dow Chemical Company), Dartford, Kent. Analar™ water was used throughout for dissolution of formulation excipients. Vitamin tablets BP (50 mg) were obtained from a local pharmacy.

Texture Analysis using a Stable Micro Systems TA.XT PlusTexture Analyser was carried out.

The preparation method consists of making the two solutions separately. Gelatine, at the required concentration, is added to water for two hours to form a dispersion and then heated to 70°C for ten minutes with gentle mixing (approximately mark 5 on a standard laboratory based heater-stirrer) to achieve dissolution. HPMC, at the required concentration, is added to water already heated to 80°C, mixed at a medium speed for thirty minutes (approximately mark 5 on a standard laboratory based heater-stirrer) and allowed to cool. Cold water is then added to achieve dissolution. When both solutions are cool they are mixed together for ten minutes. Gel formation then occurs within one hour. The gels are refrigerated prior to use.

### Experimental and Results

Please note that gelatine gels containing 2, 3 and 5% were prepared (no HPMC) and assessed. They were all considered unsuitable because without the HPMC the textures were firm and rubbery and did not appear to be suitable for our purposes. The compression tests on gelatine 2% gave an average reading of circa 150 g /5 mm as opposed to 51 g/ 5 mm with HPMC (0.4%). For 3% gelatine the result was 210g /5 mm as opposed to 110g /5 mm with HPMC (0.3%) and for 5% gelatine 480g / 5 mm as opposed to 221 g / 5 mm with HPMC (0.5%). The use of gelatine alone was not taken further as a result of these findings.

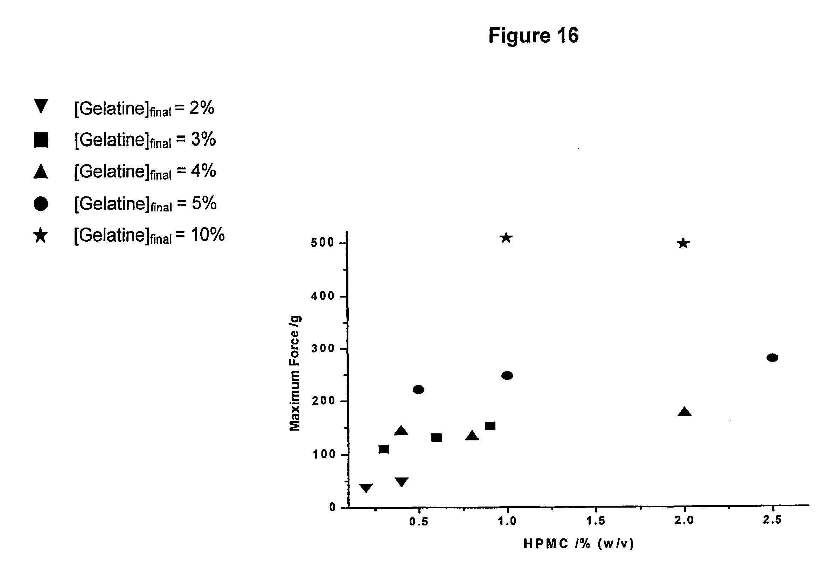

A variety of techniques were used on many different concentration combinations of gelatine and HPMC to ascertain the ideal gel in terms of comfort for patients and ease of insertion of the required tablet (ascorbic acid or vitamin C was used), the dimensions of the gel are 15 mm diameter and 7 mm depth and the dimensions of the tablet are 8 mm diameter and 3 mm depth. These included texture analysis of the gels in compression mode to monitor "softness" and breakability. Figure 16 shows an example of different concentrations of gelatine added to different concentrations of HPMC. Experimentally, concentrations of HPMC from 0.1% to 1% were tried but a concentration of 0.4% gave the best results. Additionally the concentration of gelatine used affected the softness and breakability of the gel. Concentrations of gelatine in the range 1% to 10% were also tried and a higher concentration of gelatine improved the gels tendency to split but reduced its softness. Higher compression forces were needed for the gels containing the higher concentrations of gelatine as can be seen from Figure 16. It was found that a compression force of between 50 - 120 g/ 5 mm gave the best results in terms of softness and ease of tablet insertion, below this value the gel was too soft and only semi solid and above, the gel was non-flexible and it was anticipated from tactile assessment that swallowing would be difficult

Observational analysis was used to monitor ease of handing and feel of the gels, ease of tablet insertion and the presence of syneresis (a problem found with some gel combinations). Table 7 show the results of these observations. At this stage the higher concentrations of gelatine were discarded as being too hard and so the results shown are at gelatine concentrations of 1% to 5%. HPMC concentrations were in the range of 0.1% to 2.5%. Tablet insertion and retention in the harder gels was very much easier, but these gels were too hard to swallow comfortably. The gel comprising 2% gelatine added to 0.4% HPMC was quite elastic to the touch with relative ease of tablet insertion and retention.

**Table 7: Observational analysis of gelatine/HPMC gels of different combinations. The experiments were carried out at room temperature on gels that had been left to equilibrate for one hour.**

| **Gelatine /HPMC % (w/v)** | **Ease** of **Handling** | **Syneresis** | **Feel** | **Tablet Insertion and Comments** | **Average Force /g required to compress gel 5 mm** |
|---|---|---|---|---|---|
| 1/0.1 (10:1) | Unable to pick up | no | Semi solid | No tablet insertion | - |
| 1/0.2 (5:1) | Unable to pick up | no | Semi solid | No tablet insertion | - |
| 1/0.5 (2:1) | Unable to pick up | no | Semi solid | No tablet insertion | - |
| 2/0.2 (10:1) | Able to pick up | no | Not semi solid but too soft | Tablet inserted but difficult to handle | 40.3 |
| 2/0.4 (5:1) | Able to pick up | no | Easy to handle | Table inserted without problems | 51.2 |
| 2:1 (2:1) | Unable to pick up | no | Semi solid | No tablet insertion | - |
| 3/0.3 (10:1) | Easy pick up | No | Easier to handle | Easy to insert tablet | 110 |
| 3/0.6 (5:1) | Easy pick up | Yes + | Easier to handle | Fractured on tablet insertion | 131 |
| 3/0.9 (2:1) | Easy pick up | Yes++ | Harder gel + | Easy to insert tablet, no fracture | 152 |
| 4/0.4 (10:1) | Easier pick up | No | Harder gel + | Very easy to insert tablet, no fracture | 143 |
| 4/0.8 (5:1) | Easier pick up | Yes + | Harder gel + | Very easy to insert tablet, no fracture | 132 |
| 4/2 (2:1) | Easier pick up | Yes ++ | Harder gel ++ | Very easy to insert tablet, no fracture | 174 |
| 5/0.5 (10:) | Easier pick up | Yes+ | Harder gel ++ | Very easy to insert tablet, no fracture | 221 |
| 5/1 (5:1) | Easier pick up | Yes ++ | Harder gel ++ | Very easy to insert tablet, no fracture | 247 |
| 5/2.5 (2:1) | Easier pick up | Yes ++ | Harder gel ++ | Very easy to insert tablet, no fracture | 278 |

### Conclusions

The results showed that, in terms of softness, breakability and ease of tablet insertion that a combination of 2% gelatine added to 0.4% HPMC was ideal. Lower or higher concentrations of gelatine or HPMC resulted in gels that were too soft in terms of increased breakability and difficult or impossible tablet insertion or too hard in terms of ease of swallowing.

### References

Belknap D., Seifert C. and Peterman M. (1997) "Administration of medications through enteral feeding catheters" American Journal of Critical Care 6, 382-392.
Cheeseman M. and Wallwork J. (2002) "FDA's office of fond additive safety" Food Safety Magazine: with permission of the publisher December 2002/January 2003 by the Target group. Chisaka H., Matsushima Y., Wada F. and Saeki S. (2006) "Dynamics of capsule swallowing by healthy young men and capsule transit time from the mouth to the stomach Dysphagia, 21 (4), 275-279.
Cooke I., Doods W., Dantas R., Kern M., Massey B., Shaker R. and Hogan S. (1989) "Timing of videofluoroscopic, manometric events and bolus transit time during oral and pharyngeal phases of swallowing " Dysphagia, 4, 8-15.
Deward R. and Joyce M. (2006) "Time-Dependent Rheology of Starch Thickeners and the Clinical Implications for Dysphagia Therapy", Dysphagia, 21 (4),
Finestone M., Greene-Finestone S., Wilson E. and Teasell R. (1995) "Malnutrition in stroke patients on the rehabilitation service and at follow-up: Prevalence and predictors" Archives of Physical Medicine and Rehabilitation, 76(4), 310-316.
Kikendall J.W., Friedman A.C., Oyewole M.A., Fleischer D., and Johnson L.F. (1983) "Pill-Induced oesophageal injury - case reports and review of the medical literature" Digestive disease and sciences, 28(2),
Kirkevold O. and Engedal K., (2005) Concealment (of drugs in food and beverages in nursing homes: cross sections study" British Medical Journal, 330, 20-22.Lazarus L.,
Logemann J., Rademarker A., Kahrilas P., Pajak T., Lazar R. and Halper A., (1993) "Effects of bolus volume, viscosity and repeated swallows in nonstroke subjects and stroke patients" Archives of physical medicine and rehabilitation, 74(10), 1066-1070.
Leslie P., Carding P.N. and Wilson J.A. (2003) "Investigation and management of chronic dysphagia" British Medical Journal 326, 433-436.
Logemann J. (1983) "Manual for the videofluoroscoopic study of swallowing" (2nd Ed.), Pro-Ed Inc..
McGavock H. (2005) "How drugs work. Basic pharmacology for healthcare professionals" Oxford, Radcliff publishing.
Miller J.L. and Watkin L. (1996) "The influence of bolus volume and viscosity on anterior lingual force during the oral stage of swallowing" Dysphagia 11, 117-124.
Mistry B., Samuel S., Bowden S., McCartney R. and Roberts D. (1995) "Simplifying oral drugs for patients with swallowing difficulties" Pharmaceutical Journal 254, 808-809.
Ramsey D., Smithard D. and Kalra L. (2003) "Early assessment of dysphagia and aspiration risk in acute stroke patients " Stroke 34, 1252-1257.
Shils M.E., Olson J.A., Shike M. and Ross A.C. (1995) "Modern nutrition in health and disease" IX ed. Lippincott, Williams & Wilkins, Baltimore, USA.
Smithard D., O'Neil P., England R. and Park C. (1997) "The natural history of dysphagia following a stroke" Dysphagia 12, 188-193.
Tissot E., Cornette C., Demoly P., Jacquet M., Barale F. and Capellier G. (1999) "Medication errors at the administration stage in an intensive care unit" Intensive Care Medicine 25, 353-359.
Wright D.J. (2002) "Medication administration in nursing homes" Nursing Standard 16, 33-38.

Unless otherwise indicated above, by weight means % w/w.

The foregoing broadly describes the present invention, without limitation.

## Claims

1. An edible gel composition for oral administration to a subject with difficulty in swallowing, wherein the composition is shaped and dimensioned for the acceptance of a dosage form,
wherein the gel is a water-miscible, self-supporting, flexible substance formed from a liquid, which is water, and a gelling agent, and which can be picked up and handled, and
the composition has a recess provided therein for insertion of the dosage form, which is a tablet or capsule, and wherein the recess is a cut or aperture with a length of 0.3 to 1 cm as measured across the surface of the composition, and wherein the composition comprises the gelling agent, which is gelatin, water and a lubricating agent, which is hydroxypropylmethylcellulose.

2. The composition according to any one of the preceding claims, wherein the gel composition comprises gelatin and hydroxypropylmethylcellulose (HPMC) in w/w ratio of 10:1 to 3:1.

3. The composition according to any one of the preceding claims, wherein the gel composition comprises gelatin and hydroxypropylmethylcellulose (HPMC) in w/w ratio of 6:1 to 3:1.

4. The composition according to any one of the preceding claims, wherein the gel composition comprises gelatin and hydroxypropylmethylcellulose (HPMC) in w/w ratio of about 4:1.

5. The composition according to any one of the preceding claims, wherein the gel composition consists essentially of gelatin, hydroxypropylmethylcellulose (HPMC) and water.

6. The composition according to any one of the preceding claims, wherein the recess is a cut.

7. The composition according to any one of the preceding claims, wherein the gel composition is associated with a dosage form which is a tablet or a capsule.

8. A method for preparing an edible gel composition for oral administration to a subject with difficulty in swallowing, the method comprising
providing a liquid pre-gel composition for forming a gel; and then either:
(i) solidifying the pre-gel composition in a mould such that a recess for acceptance of a dosage form is formed in the solidified edible gel composition, or
(ii) solidifying the pre-gel composition in a mould and forming a recess in the solidified gel, e.g. by cutting a recess in the gel,
wherein the edible gel composition comprises the gelling agent, which is gelatin, water and a lubricating agent, which is hydroxypropylmethylcellulose,
and the recess in the edible gel composition is for insertion of the dosage form, wherein the dosage form is a tablet or capsule and wherein the recess is a cut or aperture with a length of about 0.3 to 1 cm as measured across the surface of the composition.

9. A dosage form for use in a method of treating a subject with difficulty in swallowing and optionally an another disorder, the method comprising administering a dosage form to the subject with difficulty in swallowing, comprising:
providing a composition as defined in any one of claims 1 to 7 having a dosage form associated with the composition, wherein the dosage form is present in the recess of the composition, the dosage form is a tablet or capsule, and the dosage form being for the treatment of the difficulty in swallowing and/or the other disorder,
administering the composition orally to the subject.

10. The dosage form of claim 9, wherein the other disorder is a disorder associated with the difficulty in swallowing selected from blunt throat injury, surgery-caused impairment, stroke, multiple sclerosis, Asperger syndrome, esophageal cancer, laryngeal cancer, chagus disease, celiac, cystic fibrosis, Huntington's disease, Niemann-Pick disease, neurological conditions such as amyotrophic lateral sclerosis, Alzheimer's and Parkinson's disease, obesity, Riley- Day syndrome, high cholesterol, corn allergies and corn sensitivities, sclerodenna, and diabetes.

11. A package containing a composition as defined in any one of claims 1 to 7, wherein at least part of the edible composition is exposed, and the recess is present on the exposed part of the edible composition.

12. A package according to claim 11, wherein the package comprises:
(i) a base having one or more indentations containing a gel composition as defined in any one of claims 1 to 7, the composition having a recess therein for the insertion of a dosage form; and
(ii) a removable cover, such that when the cover is removed, the recess is exposed to allow insertion of a dosage form into the recess.

## Patentansprüche

1. Essbare Gelzusammensetzung für die orale Verabreichung an ein Subjekt mit Schluckschwierigkeiten, wobei die Zusammensetzung für die Annahme einer Darreichungsform geformt und dimensioniert ist,
wobei das Gel eine wasservermischbare, selbsttragende flexible Substanz ist, gebildet aus einer Flüssigkeit, die Wasser ist, und einem Geliermittel, und welche aufgehoben und angefasst werden kann, und
wobei die Zusammensetzung mit einer Vertiefung versehen ist zum Einführen in die Darreichungsform, welche eine Tablette oder eine Kapsel ist, und wobei die Vertiefung ein Schnitt oder eine Öffnung ist mit einer Länge von 0,3 bis 1 cm, gemessen quer über die Oberfläche der Zusammensetzung, und wobei die Zusammensetzung das Geliermittel umfasst, welches Gelatine ist, Wasser und ein Schmiermittel, welches Hydroxypropylmethylcellulose ist.

2. Zusammensetzung gemäß irgendeinem der vorhergehenden Ansprüche, wobei die Gelzusammensetzung Gelatine und Hydroxypropylmethylcellulose (HPMC) in einem Gewichtsverhältnis von 10:1 bis 3:1 umfasst.

3. Zusammensetzung gemäß irgendeinem der vorhergehenden Ansprüche, wobei die Gelzusammensetzung Gelatine und Hydroxypropylmethylcellulose (HPMC) in einem Gewichtsverhältnis von 6:1 bis 3:1 umfasst.

4. Zusammensetzung gemäß irgendeinem der vorhergehenden Ansprüche, wobei die Gelzusammensetzung Gelatine und Hydroxypropylmethylcellulose (HPMC) in einem Gewichtsverhältnis von etwa 4:1 umfasst.

5. Zusammensetzung gemäß irgendeinem der vorhergehenden Ansprüche, wobei die Gelzusammensetzung im Wesentlichen aus Gelatine, Hydroxypropylmethylcellulose (HPMC) und Wasser besteht.

6. Zusammensetzung gemäß irgendeinem der vorhergehenden Ansprüche, wobei die Vertiefung ein Schnitt ist.

7. Zusammensetzung gemäß irgendeinem der vorhergehenden Ansprüche, wobei die Gelzusammensetzung in Zusammenhang steht mit einer Darreichungsform, die eine Tablette oder eine Kapsel ist.

8. Herstellungsverfahren für eine essbare Gelzusammensetzung für die orale Verabreichung an ein Subjekt mit Schluckschwierigkeiten, das Verfahren umfassend
Bereitstellen einer flüssigen Vor-Gelzusammensetzung zum Bilden eines Gels; und dann entweder:
(i) Aushärten der Vor-Gelzusammensetzung in einer Form, so dass eine Vertiefung für die Aufnahme einer Darreichungsform in der ausgehärteten essbaren Gelzusammensetzung gebildet wird, oder
(ii) Aushärten der Vor-Gelzusammensetzung in einer Form und Bilden einer Vertiefung im ausgehärteten Gel, z.B. durch Schneiden einer Vertiefung in das Gel,
wobei die essbare Gelzusammensetzung das Geliermittel umfasst, welches Gelatine ist, Wasser und ein Schmiermittel, welches Hydroxypropylmethylcellulose ist,
und die Vertiefung in der essbaren Gelzusammensetzung für die Einführung in die Darreichungsform ist, wobei die Darreichungsform eine Tablette oder Kapsel ist und wobei die Vertiefung ein Schnitt oder eine Öffnung ist mit einer Länge von 0,3 bis 1 cm ist, gemessen quer über die Oberfläche der Zusammensetzung.

9. Darreichungsform für die Verwendung in einem Verfahren zum Behandeln eines Subjekts mit Schluckschwierigkeiten und wahlweise einer weiteren Krankheit, das Verfahren umfassend Verabreichen einer Darreichungsform an das Subjekt mit Schluckschwierigkeiten, umfassend
Bereitstellen einer Zusammensetzung, wie in irgendeinem der Ansprüche 1 bis 7 definiert, welche eine mit der Zusammensetzung in Verbindung stehende Darreichungsform hat, wobei die Darreichungsform in der Vertiefung der Zusammensetzung vorliegt, die Darreichungsform eine Tablette oder Kapsel ist und die Darreichungsform für die Behandlung der Schluckschwierigkeiten und/oder der weiteren Krankheit ist,
orale Verabreichung der Zusammensetzung an das Subjekt.

10. Darreichungsform gemäß Anspruch 9, wobei die weitere Krankheit eine Krankheit ist, in Verbindung mit der Schluckschwierigkeit, ausgewählt aus stumpfe Rachenverletzung, operationsausgelöste Einschränkung, Schlaganfall, multiple Sklerose, Asperger-Syndrom, Speiseröhrenkrebs, Kehlkopfkrebs, Chagas-Krankheit, Zöliakie, Mukoviszidose, Huntington-Krankheit, Niemann-Pick-Krankheit, neurologische Krankheiten, wie amyotrophe Lateralsklerose, Alzheimer- und Parkinson-Krankheit, Übergewicht, Riley-Day-Syndrom, hohes Cholesterin, Maisallergien und Maisüberempfindlichkeiten, Sklerodermie und Diabetes.

11. Paket, umfassend eine Zusammensetzung, wie in irgendeinem der Ansprüche 1 bis 7 definiert, wobei mindestens ein Teil der essbaren Zusammensetzung freiliegt und die Vertiefung sich am freigelegten Teil der essbaren Zusammensetzung befindet.

12. Paket gemäß Anspruch 11, wobei das Paket umfasst
(i) eine Basis mit einer oder mehreren Einbuchtungen, enthaltend eine Gelzusammensetzung, wie in irgendeinem der Ansprüche 1 bis 7 definiert, wobei die Zusammensetzung eine Vertiefung darin aufweist für das Einführen einer Darreichungsform; und
(ii) eine entfernbare Abdeckung, so dass, wird die Abdeckung entfernt, die Vertiefung freigelegt wird, um ein Einführen einer Darreichungsform in die Vertiefung zu ermöglichen.

## Revendications

1. Une composition de gel comestible pour une administration orale à un sujet ayant des difficultés de déglutition, où la composition est façonnée et dimensionnée pour l'acceptation d'une forme posologique,
où le gel est une substance miscible à l'eau, autonome et flexible formée à partir d'un liquide, qui est de l'eau, et un agent gélifiant, et qui peut être saisi et manipulé, et
la composition possède un évidement fourni dans celle-ci pour insertion de la forme posologique, qui est un comprimé ou une capsule, et où l'évidement est une découpe ou une ouverture d'une longueur de 0,3 à 1 cm telle que mesurée sur la surface de la composition, et où la composition contient l'agent gélifiant, qui est gélatine, eau et un agent lubrifiant, qui est hydroxypropylméthylcellulose.

2. La composition selon l'une quelconque des revendications précédentes, où la composition de gel contient gélatine et hydroxypropylméthylcellulose (HPMC) dans un rapport poids/poids de 10:1 à 3:1.

3. La composition selon l'une quelconque des revendications précédentes, où la composition de gel contient gélatine et hydroxypropylméthylcellulose (HPMC) dans un rapport poids/poids de 6:1 à 3:1.

4. La composition selon l'une quelconque des revendications précédentes, où la composition de gel contient gélatine et hydroxypropylméthylcellulose (HPMC) dans un rapport poids/poids d'environ 4:1.

5. La composition selon l'une quelconque des revendications précédentes, où la composition de gel se compose essentiellement de gélatine, hydroxypropylméthylcellulose (HPMC) et eau.

6. La composition selon l'une quelconque des revendications précédentes, où l'évidement est une découpe.

7. La composition selon l'une quelconque des revendications précédentes, où la composition de gel est associée à une forme posologique qui est un comprimé ou une capsule.

8. Un procédé de préparation d'une composition de gel comestible pour une administration orale à un sujet ayant des difficultés de déglutition, le procédé comprenant
la fourniture d'une pré-composition de gel liquide destiné à la formation d'un gel, et ensuite soit :
(i) la solidification de la pré-composition de gel dans un moule de sorte qu'un évidement pour l'acceptation d'une forme posologique soit formé dans la composition de gel comestible solidifiée, ou
(ii) la solidification de la pré-composition de gel dans un moule et la formation d'un évidement dans le gel solidifié, par exemple par le découpage d'un évidement dans le gel,
où la composition de gel comestible contient l'agent gélifiant, qui est gélatine, eau et un agent lubrifiant, qui est hydroxypropylméthylcellulose,
et l'évidement dans la composition de gel comestible est destiné à l'insertion de la forme posologique, où la forme posologique est un comprimé ou une capsule et où l'évidement est une découpe ou une ouverture d'une longueur d'environ 0,3 à 1 cm telle que mesurée sur la surface de la composition.

9. Une forme posologique destinée à une utilisation dans un procédé de traitement d'un sujet ayant des difficultés de déglutition et éventuellement un autre trouble, le procédé comprenant l'administration d'une forme posologique au sujet ayant des difficultés de déglutition, comprenant :
la fourniture d'une composition telle que définie dans l'une quelconque des revendications 1 à 7 possédant une forme posologique associée à la composition, où la forme posologique est présente dans l'évidement de la composition, la forme posologique est un comprimé ou une capsule, et la forme posologique étant destinée au traitement des difficultés de déglutition et/ou de l'autre trouble,
l'administration orale de la composition au sujet.

10. La forme posologique selon la revendication 9, où l'autre trouble est un trouble associé à des difficultés de déglutition sélectionné parmi blessure contendante de la gorge, lésion due à une opération chirurgicale, attaque vasculaire cérébrale, sclérose en plaques, syndrome d'Asperger, cancer de l'oesophage, cancer du larynx, maladie de Chagas, maladie coeliaque, fibrose kystique, maladie de Huntington, maladie de Niemann-Pick, troubles neurologiques tels que sclérose latérale amyotrophique, maladie d'Alzheimer et maladie de Parkinson, obésité, syndrome de Riley-Day, cholestérol élevé, allergies au maïs et sensibilités au maïs, sclérodermie et diabète.

11. Un conditionnement contenant une composition telle que définie dans l'une quelconque des revendications 1 à 7, où au moins une partie de la composition comestible est exposée et l'évidement est présent sur la partie exposée de la composition comestible.

12. Un conditionnement selon la revendication 11, où le conditionnement contient :
(i) une base possédant une ou plusieurs indentations contenant une composition de gel selon l'une quelconque des revendications 1 à 7, la composition possédant un évidement dans celle-ci pour l'insertion d'une forme posologique, et
(ii) a couvercle amovible, tel que, lorsque le couvercle est retiré, l'évidement est exposé de façon à permettre l'insertion d'une forme posologique dans l'évidement.
